# EUROPEAN PATENT SPECIFICATION

(11) **EP 2 086 998 B1**
(45) Date of publication and mention of the grant of the patent: **07.12.2011**
(21) Application number: 07821216.4
(22) Date of filing: 11.10.2007
(51) Int. Cl.: C07K 14/00, A61K 38/04, C12N 15/12

(54) **AMINO ACID SEQUENCES THAT BIND TO SERUM PROTEINS IN A MANNER THAT IS ESSENTIALLY INDEPENDENT OF THE PH, COMPOUNDS COMPRISING THE SAME, AND USE THEREOF**
IM WESENTLICHEN PH-WERT-UNABHÄNGIGERWEISE AN SERUMPROTEINE BINDENDE AMINOSÄURESEQUENZEN, VERBINDUNGEN, DIE DIESE ENTHALTEN, UND DEREN VERWENDUNG
SEQUENCES D'ACIDES AMINES SE LIANT A DES PROTEINES SERIQUES ESSENTIELLEMENT INDEPENDAMMENT DU PH, COMPOSES COMPRENANT CELLES ET UTILISATIONS CORRESPONDANTES

(30) Priority: 11.10.2006 US 850774 P
(43) Date of publication of application: 12.08.2009
(73) Proprietor: Ablynx N.V., 9052 Ghent-Zwijnaarde (BE)
(72) Inventor: LASTERS, Ignace Joseph Isabella, B-2018 Antwerpen (BE); HOOGENBOOM, Hendricus Renerus Jacobus Mattheus, NL-6214 AE Maastricht (NL)
(74) Representative: HOFFMANN EITLE
(86) International application number: PCT/EP2007/060849
(87) International publication number: WO 2008/043821

(56) References cited:
- WO-A-2004/003019
- WO-A-2004/040153
- WO-A-2004/041863
- WO-A-2004/041867
- WO-A-2004/062551
- WO-A-2005/044858
- WO-A-2006/059206

## Description

### Field of the invention

The present invention relates to immunoglobulin variable domain sequences as defined in claim 1 that are capable of binding to serum proteins such as serum albumin; to compounds, proteins and polypeptides comprising or essentially consisting of such amino acid sequences; to nucleic acids that encode such amino acid sequences, proteins or polypeptides; to compositions, and in particular pharmaceutical compositions, that comprise such amino acid sequences, proteins and polypeptides; and to uses of such amino acid sequences, proteins and polypeptides.

In particular, the invention relates to such immunoglobulin variable domain sequences (and compounds comprising the same), that bind to a serum protein in a manner that, at physiological values of the pH, is essentially independent of the pH (as defined herein).

According to the invention, it has been found that such immunoglobulin variable domain sequences (as well as compounds that comprise at least one such immunoglobulin variable domain sequence, as further described herein) have a favourable (i.e. longer) half-life in circulation, compared to amino acid sequences that bind to the same serum protein that is not essentially independent of the pH.

Other aspects, embodiments, advantages and applications of the invention will become clear from the further description herein.

### Background of the invention

Amino acid sequences that are capable of binding to human serum proteins such as human serum albumin and uses thereof in polypeptide constructs in order to increase the half-life of therapeutically relevant proteins and polypeptides are known in the art.

For example, WO 91/01743, WO 01/45746 and WO 02/076489 describe peptide moieties binding to serum albumin that can be fused to therapeutic proteins and other therapeutic compounds and entities in order to increase the half-life thereof. However, these peptide moieties are of bacterial or synthetic origin, which is less preferred for use in therapeutics.

WO 04/041865 by Ablynx N.V. describes Nanobodies® directed against serum albumin (and in particular against human serum albumin) that can be linked to other proteins (such as one or more other Nanobodies® directed against a desired target) in order to increase the half-life of said protein.

The neonatal Fc receptor (FcRn), also termed "Brambell receptor", is involved in prolonging the life-span of albumin in circulation (see Chaudhury et al., The Journal of Experimental Medicine, vol. 3, no. 197, 315-322 (2003)). The FcRn receptor is an integral membrane glycoprotein consisting of a soluble light chain consisting of β2-microglobulin, noncovalently bound to a 43 kD α chain with three extracellular domains, a transmembrane region and a cytoplasmic tail of about 50 amino acids. The cytoplasmic tail contains a dinucleotide motif-based endocytosis signal implicated in the internalization of the receptor. The α chain is a member of the nonclassical MHC I family of proteins. The β2m association with the α chain is critical for correct folding of FcRn and exiting the endoplasmic reticulum for routing to endosomes and the cell surface.

The overall structure of FcRn is similar to that of class I molecules. The α-1 and α-2 regions resemble a platform composed of eight antiparallel β strands forming a single β-sheet topped by two antiparallel α-helices very closely resembling the peptide cleft in MHC I molecules. Owning to an overall repositioning of the α-1 helix and bending of the C-terminal portion of the α-2 helix due to a break in the helix introduced by the presence of Pro162, the FcRn helices are considerably closer together, occluding peptide binding. The side chain of Arg164 of FcRn also occludes the potential interaction of the peptide N-terminus with the MHC pocket. Further, salt bridge and hydrophobic interaction between the α-1 and α-2 helices may also contribute to the groove closure.

FcRn therefore, does not participate in antigen presentation, and the peptide cleft is empty.

FcRn binds and transports IgG across the placental syncytiotrophoblast from maternal circulation to fetal circulation and protects IgG from degradation in adults. In addition to homeostasis, FcRn controls transcytosis of IgG in tissues. FcRn is localized in epithelial cells, endothelial cells and hepatocytes.

According to Chaudhury et al. (supra), albumin binds FcRn to form a tri-molecular complex with IgG. Both albumin and IgG bind noncooperatively to distinct sites on FcRn. Binding of human FcRn to Sepharose-HSA and Sepharose-hlgG was pH dependent, being maximal at pH 5.0 and approaching nil at pH 7.0 through pH 8. The observation that FcRn binds albumin in the same pH dependent fashion as it binds IgG suggests that the mechanism by which albumin interacts with FcRn and thus is protected from degradation is identical to that of IgG, and mediated via a similarly pH-sensitive interaction with FcRn. Using SPR to measure the capacity of individual HSA domains to bind immobilized soluble hFcRn, Chaudhury showed that FcRn and albumin interact via the D-III domaine of albumin in a pH-dependent manner, on a site distinct from the IgG binding site (Chaudhury, PhD dissertation, see http://www.andersonlab.com/biosketchCC.htm; Chaudhury et al. Biochemistry, ASAP Article 10.1021/bi052628y S0006-2960(05)02628-0 (Web release date: March 22, 2006); see also Chaudhury, Biochemistry, 2006, vol. 45, 4983-4990).

A major disadvantage of albumin binders known in the art is their limited half-life in vivo in primates. In mice, the natural half-life of serum albumin is approximately 2 days, and different serum albumin binders have been shown to exhibit a comparable half-life, i.e. approximately 2 days. However, to the extent that known serum albumin binders have been tested in primates (i.e. of the genus *Macaca*, such as rhesus monkeys and cynomologus monkeys), they have exhibited a serum half-life of approximately 3 days, Reference is for example made to the data on the so-called "AlbudAb's™" (AlbudAb™ is a trademark of Domantis Ltd., Cambridge, UK) by Dr. Lucy Holt of Domantis Ltd. in the presentation "Tailoring Human Domain Antibodies for Best Practices" given on June 1, 2006 during the IBC Conference "Antibodies and Beyond" on June 1, 2006. In other words, the serum albumin binders for which half-life data in primates is known in the art are deficient in that they exhibit short serum half-lives in primates in vivo. These half-lives are considerably shorter than the natural half-live of serum albumin in these animals, e.g. 25% thereof.

Many therapeutics, in particular biologicals (i.e. peptide or polypeptide drugs, polynucleotides, etc.) suffer from inadequate serum half-lives in vivo. This necessitates the administration of such therapeutics at high frequencies and/or higher doses, or the use of sustained release formulations, in order to maintain the serum levels necessary for therapeutic effects. Frequent systemic administration of drugs is associated with considerable negative side effects. For example, frequent, e.g. daily, systemic injections represent a considerable discomfort to the subject, and pose a high risk of administration related infections, and may require hospitalization or frequent visits to the hospital, in particular when the therapeutic is to be administered intravenously. Moreover, in long term treatments daily intravenous injections can also lead to considerable side effects of tissue scarring and vascular pathologies caused by the repeated puncturing of vessels. Similar problems are known for all frequent systemic administrations of therapeutics, like, for example, the administration of insulin to diabetics, or interferon drugs in patients suffering from multiple sclerosis. All these factors lead to a decreased patient compliance and increased costs for the health system.

Therefore, there is a need for means to increase the serum half-life of therapeutics in primates, in particular in humans.

### Summary of the invention

The present invention solves this need by providing immunoglobulin variable domain sequences (and compounds comprising the same), that bind to a serum protein in a manner that, at physiological values of the pH, is essentially independent of the pH (as described herein). In the following description reference to an "amino acid sequence" of the invention is meant to relate to an "immunoglobulin variable domain".

The serum protein to which the amino acid sequences of the invention bind (or under physiological conditions can bind) is a serum protein that is subject to recycling or a recycling mechanism in the human or animal body in which said serum protein naturally occurs, i.e.

the serum protein to which the amino acid sequences of the invention bind (or under physiological conditions can bind) is chosen from the group consisting of: serum albumin, immunoglobulins such as IgG and transferrin. According to a preferred, but non-limiting embodiment, the amino acid sequences of the invention bind to serum albumin.

The serum protein is preferably a human serum protein. However, it should be understood that according to some specific but non-limiting aspects of the invention, the amino acid sequences of the invention may be cross-reactive with the corresponding (i.e. orthologous) serum protein from at least another species of mammal, such as mouse, rat, rabbit, dog or primate. In particular, according to these aspects, the amino acid sequences of the invention may be cross-reactive with the corresponding (i.e. orthologous) serum protein from at least another species of primate, as further described herein.

By binding that is "*essentially independent of the pH*" is generally meant herein that the association constant (K_{A}) of the amino acid sequence with respect to the serum protein (such as serum albumin) at the pH value(s) that occur in a cell of an animal or human body (as further described herein) is at least 50%, even more preferably at least 60%, such as even more preferably at least 70%, such as at least 80% or 90% or more (or even more than 100%, such as more than 110%, more than 120% or even 130% or more, or even more than 150%, or even more than 200%) of the association constant (K_{A}) of the amino acid sequence with respect to the same serum protein at the pH value(s) that occur outside said cell. Alternatively, by binding that is "*essentially independent of the pH*" is generally meant herein that the k_{off} rate (measured by Biacore - see e.g. Experiment 2) of the amino acid sequence with respect to the serum protein (such as serum albumin) at the pH value(s) that occur in a cell of an animal or human body (as e.g. further described herein, e.g. pH around 5.5, e.g. 5.3 to 5.7) is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more, or even more than 150%, or even more than 200%) of the k_{off} rate of the amino acid sequence with respect to the same serum protein at the pH value(s) that occur outside said cell, e.g. pH 7.2 to 7.4.

By "*the pH value(s) that occur in a cell of an animal or human body"* is meant the pH value(s) that may occur inside a cell, and in particular inside a cell that is involved in the recycling of the serum protein. In particular, by "*the pH value(s) that occur in a cell of an animal or human body*" is meant the pH value(s) that may occur inside a (sub)cellular compartment or vesicle that is involved in recycling of the serum protein (e.g. as a result of pinocytosis, endocytosis, transcytosis, exocytosis and phagocytosis or a similar mechanism of uptake or internalization into said cell), such as an endosome, lysosome or pinosome.

For example, the cell may be a cell that contains or expresses the FcRn receptor, in particular when the amino acid sequence of the invention is directed against a serum protein that binds to FcRn. As will become clear from the further description herein, such cells are involved in recycling of certain serum proteins that can bind to FcRn, such as serum albumin and immunoglobulins such as IgG. Alternatively, for example and without limitation, the cell may be a cell that contains or expresses the transferrin-receptor, in particular when the amino acid sequence of the invention is directed against transferrin.

By "*the pH value(s) that occur outside said cell*" is generally meant the pH value(s) that may occur inside the body of the human or animal in which said cell is present, but outside said cell, such as at the cell surface or in the immediate surroundings or near vicinity of said cell. In particular, by "*the pH value(s) that occur outside said cell*" is meant the pH value(s) that may occur in the circulation of the human or animal body in which said cell is present, such as in the blood(stream) or in the lymphatic system.

By a "*physiological pH value*" is generally meant any pH value that naturally occurs in a human or animal body (i.e. either of a healthy animal or human or of an animal or human that is suffering from a disease or disorder). Such physiological pH values will be clear to the skilled person. It will also be clear to the skilled person that different physiological pH values may occur in different parts, tissues, fluids (such as blood or lymph fluid), cells, subcellular compartments, etc..

The amino acid sequences are such that they bind to the serum protein (such as serum albumin) at a pH value in the range of 6.5 to 5.5 (such as 6.5, 6.4, 6.3, 6.2, 6.1, 6.0, 5.9, 5.8, 5.7 or 5.6) with an association constant (K_{A}) that is at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at a pH in the range of 7.2 to 7.4 (such as 7.2, 7.3 or 7.4).

Also described are amino acid sequences that bind to the serum protein (such as serum albumin) at a pH value in the range of 5.5 to 4.5 (such as 5.5, 5.4, 5.3, 5.2, 5.1, 5.0, 4.9, 4.8, 4.7 or 4.6) with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at a pH in the range of 6.8 to 7.4 (such as 6.8, 6.9, 7.0, 7.1, 7.2, 7.3 or 7.4).

Also described are amino acid sequences bind to the serum protein (such as serum albumin) at a pH value in the range of 5.5 to 4.5 (such as 5.5, 5.4, 5.3, 5.2, 5.1, 5.0, 4.9, 4.8, 4.7 or 4.6) with an off-rate (k_{off} rate) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120%, more than 150%, more than 170%, more than 200% or even 250% or more) of the k_{off} rate of the amino acid sequence with respect to the same serum protein at a pH in the range of 6.8 to 7.4 (such as 6.8, 6.9, 7.0, 7.1, 7.2, 7.3 or 7.4).

In another embodiment, an amino acid sequence of the invention is such that said amino acid sequence binds to the serum protein (such as serum albumin) at a pH val ue in the range of 5.5 to 4.5 (such as 5.5, 5.4, 5.3, 5.2, 5.1, 5.0, 4.9, 4.8, 4.7 or 4.6) with a k_{off} rate that is 50% more or less (i.e. between 50 to 150%) of the k_{off} rate of the amino acid sequence with respect to the same serum protein at a pH in the range of 6.8 to 7.4 (such as 6.8, 6.9, 7.0, 7.1, 7.2, 7.3 or 7.4). More preferably the k_{off} rate for said amino acid sequence (e.g. a multivalent Nanobody of the invention, e.g. a Nanobody of the invention which binds monovalently to serum albumin,e g. human serum albumin, and monovalently or multivalently to a target protein) at a pH in the range of 6.8 to 7.4 (such as 6.8, 6.9, 7.0, 7.1, 7.2, 7.3 or 7.4) is 40% more or less of the k_{off} rate of said amino acid sequence at a pH in the range of 5.5 to 4.5 (such as 5.5, 5.4, 5.3, 5.2, 5.1, 5.0, 4.9, 4.8, 4.7 or 4.6).

More preferably the k_{off} rate for said amino acid sequence (e.g. a multivalent Nanobody of the invention, e.g. a Nanobody of the invention which binds monovalently to serum albumin,e g. human serum albumin, and monovalently or multivalently to a target protein) at a pH in the range of 6.8 to 7.4 (such as 6.8, 6.9, 7.0, 7.1, 7.2, 7.3 or 7.4) is 30%, more preferably 20%, more preferably 10%, more or less of the, k_{off} rate of said amino acid sequence at a pH in the range of 5.5 to 4.5 (such as 5.5, 5.4, 5.3, 5.2, 5.1, 5.0, 4.9, 4.8, 4.7 or 4.6).

The association constant may be the actual or apparent association constant, as will be clear to the skilled person. Methods for determining the association constant at a certain pH value will be clear to the skilled person, and for example include the techniques mentioned herein. Optionally, as will also be clear to the skilled person, the (actual or apparent) association constant may be calculated on the basis of the (actual or apparent) dissociation constant, by means of the relationship [K_{A} = 1/K_{D}]. For this purpose, methods for determining the dissociation constant at a certain pH value will be clear to the skilled person, and for.example include the techniques mentioned herein.

The affinity denotes the strength or stability of a molecular interaction. The affinity is commonly given as by the Kd, or dissociation constant, which has units of mol/liter, noted in brief as M. The affinity can also be expressed as an association constant, Ka which equals 1/Kd and has units of (mol/liter)⁻¹, in brief M⁻¹. Throughout this document we will express the stability of molecular interaction by its Kd value. But it should be understood that in view of the relation Ka =1/Kd, specifying the strength of molecular interaction by its Kd value, automatically specifies also the Ka value. The Kd characterizes the strength of a molecular interaction also in a thermodynamic sense as it is related to the free energy (DG) of binding by the well known relation DG=RT.In(Kd) (equivalently DG=-RT.In(Ka)), where R equals the gas constant, T equals the absolute temperature and In denotes the natural logarithm. The Kd of meaningful biological complexes are typically in the range of 10⁻¹⁰M (0.1 nM) to 10⁻⁵M (10000nM). The stronger an interaction is, the lower is its Kd.

Kd can also be expressed as the ratio of the dissociation rate constant of a complex, denoted as k_{off}, to the rate of its association, denoted kₒₙ. In other words Kd=k_{off}/kₒₙ. Clearly Ka = kₒₙ/k_{off}. The off-rate k_{off} has units s⁻¹ (where s is the SI unit notation of second). The on-rate kₒₙ has units M⁻¹s⁻¹. The on-rate may vary between 10² M⁻¹s⁻¹ to about 10⁷ M⁻¹s⁻¹, approaching the diffusion-limited association rate constant for bimolecular interactions. The off-rate is related to the half-life of a given molecular interaction by the relation t_{1/2}=ln(2)/k_{off}. The off-rate may vary between 10⁻⁶ s⁻¹ (near irreversible complex with a t_{1/2} of multiple days) to 1s⁻¹ (t_{1/2}=0.69 s).

The affinity of a molecular interaction between two molecules can be measured via different techniques such the well the known surface plasmon resonance (SPR) biosensor technique (e.g. Ober et al., Intern. Immunology, 13, 1551-1559, 2001 used a Biacore 3000 SPR biosensor to study the affinity of albumin for FcRn under various pH conditions) where one molecule is immobilized on the biosensor chip and the other molecule is passed over the immobilized molecule under flow conditions yielding kₒₙ, k_{off} measurements and hence Kd (or Ka) values.

It should be noted that the measured Kd corresponds to an apparent Kd if the measuring process somehow influences the intrinsic binding affinity of the implied molecules for example by artifacts related to the coating on the biosensor of one molecule. Also, an apparent Kd may be measured if one molecule contains more than one recognition sites for the other molecule. In such situation the measured affinity may be affected by the avidity of the interaction by the two molecules. For example, SPR experiments with immobilized human FcRn show a significantly higher affinity (avidity) for human IgG as compared to the affinity of the FcRn interaction with immobilized IgG paralleling the 2:1 stoichiometry of the FcRn-IgG interaction (Sánchez et al., Biochemistry, 38, 9471-9476, 1999).

Another approach that may be used to assess affinity is the 2-step ELISA (Enzyme-Linked Immunosorbent Assay) procedure of Friguet et al. (J. Immunol. Methods, 77, 305-19, 1985). This method establishes a solution phase binding equilibrium measurement and avoids possible artifacts relating to adsorption of one of the molecules on a support such as plastic.

For example Nguyen et al. (Protein Eng Des Sel., 19, 291-297, 2006) have recently measured the affinity for albumin of Fab constructs using the Friguet assay. However, the accurate measurement of Kd may be quite labor-intensive and as consequence, often apparent Kd values are determined to assess the binding strength of two molecules. It should be noted that as long all measurements are made in a consistent way (e.g. keeping the assay conditions unchanged) apparent Kd measurements can be used as an approximation of the true Kd and hence in the present document Kd and apparent Kd should be treated with equal importance or relevance.

Finally, it should be noted that in many situations the experienced scientist may judge it to be convenient to determine the binding affinity relative to some reference molecule. For example, to assess the binding strength between molecules A and B, one may e.g. use a reference molecule C that is known to bind to B and that is suitably labeled with a fluorophore or chromophore group or other chemical moiety, such as biotin for easy detection in an ELISA or FACS (Fluorescent activated cell sorting) or other format (the fluorophore for fluorescence detection, the chromophore for light absorption detection, the biotin for streptavidin-mediated ELISA detection). Typically, the reference molecule C is kept at a fixed concentration and the concentration of B is varied for a given concentration or amount of B. As a result an IC50 value is obtained corresponding to the concentration of A at which the signal measured for C in absence of A is halved. Provided Kd_{ref}, the Kd of the reference molecule, is known, as well as the total concentration c_{ref} of the reference molecule, the apparent Kd for the interaction A-B can be obtained from following formula: Kd=IC50/(1+c_{ref}/Kd_{ref}). Note that if c_{ref} << Kd_{ref}, Kd = IC50. Provided one performs the IC50 measurement in a consistent way (e.g. keeping c_{ref} fixed), the strength or stability of a molecular interaction can be assessed by the IC50 and this measurement is judged as equivalent to Kd or to apparent Kd throughout this text.

According to the invention, it has been found that amino acid sequences that bind to a serum protein in a manner that is essentially independent of the pH (as well as compounds that comprise at least one such amino acid sequence, as further described herein), have a favourable (i.e. longer) half-life in circulation than amino acid sequences that bind to said serum protein that is not essentially independent of the pH. Without being limited to any mechanism or explanation, it is assumed that this independence of the pH will provide essentially a maximal amount of the amino acid sequence to be recycled due to the fact that during changes in the pH during the recycling process, the amino acid sequence retains its binding activity for the serum protein. If the interaction of the amino acid sequence with the serum protein is sensitive to the pH, this will lead to a reduced or loss of interaction and therefore the amino acid sequence will detach from the recycling serum protein and be targeted for degradation in the endosomal and lysosomal compartments.

In particular, the amino acid sequences of the invention (as well as compounds comprising the same, as defined herein) may be such that they bind to or otherwise associate with a serum protein (such as serum albumin) in such a way that, when the amino acid sequence is bound to or otherwise associated with said serum protein molecule (such as serum albumin) in a primate, it exhibits a serum half-life of at least about 50% (such as about 50% to 70%), preferably at least 60% (such as about 60% to 80%) or preferably at least 70% (such as about 70% to 90%), more preferably at least about 80% (such as about 80% to 90%) or preferably at least about 90% of the natural half-life of serum proteins such as serum albumin in said primate. For example, the amino acid sequences of the invention may bind to or otherwise associate with human serum proteins such as serum albumin in such a way that, when the amino acid sequences are bound to or otherwise associated with a human serum protein such as serum albumin, the amino acid sequences exhibit a serum half-life in human of at least about 50% (such as about 50% to 70%), preferably at least 60% (such as about 60% to 80%) or preferably at least 70% (such as about 70% to 90%), more preferably at least about 80% (such as about 80% to 90%) or preferably at least about 90% of the natural half-life of said serum protein (such as human serum albumin). Also, preferably, the amino acid sequences of the invention bind to said serum protein (such as human serum albumin) with a dissociation constant (K_{D}) and/or with a binding affinity (K_{A}) that is as defined herein. In man, the half-life of serum albumin is about 19 days (Peters T (1996) All About Albumin. Academic Press, San Diego).

This in vivo half-life in primates makes the amino acid sequences of the invention ideal candidates to prolong the serum half-life of therapeutics attached thereto. A long serum half-life of the combined amino acid sequence and therapeutics according to the invention in turn allows for reduced frequencies of administration and/or reduced amount to be administered, bringing about significant benefits for the subject to be treated.

The invention therefore also relates to compounds of the invention that comprise such an amino acid sequence and that have a half-life in human that is at least 80%, more preferably at least 90%, such as 95% or more or essentially the same as the half-life in human of the amino acid sequence present in said compound.

In one specific aspect, the amino acid sequences of the invention may be such that they are cross-reactive with the corresponding (i.e. orthologous) serum protein (such as serum albumin) from at least one further species of primate, and in particular with the corresponding (i.e. orthologous) serum protein from at least one species of primate that is chosen from the group consisting of monkeys from the genus *Macaca* (such as, and in particular, cynomologus monkeys (*Macaca fuscicularis*) and/or rhesus monkeys (*Macaca. mulatta*)) and baboon (*Papio ursinus*). Preferably, such cross-reactive amino acid sequences are further such that they exhibit a serum half-life in said primate of at least about 50% (such as about 50°% to 70%), preferably at least 60% (such as about 60% to 80%) or preferably at least 70% (such as about 70% to 90%), more preferably at least about 80% (such as about 80% to 90%) or preferably at least about 90% of the natural half-life of the corresponding (i.e. orthologous) serum protein (such as serum albumin) in said primate. Such amino acid sequences of the invention also preferably bind to the corresponding (i.e. orthologous) serum protein (such as serum albumin) from said primate with a dissociation constant (K_{D}) and/or with a binding affinity (K_{A}) that is as defined herein.

The invention also relates to compounds of the invention that comprise at least one amino acid sequence of the invention and that have a half-life in human and/or in said at least one species of primate that is at least 80%, more preferably at least 90%, such as 95% or more or essentially the same as the half-life in human and/or said species of primate, respectively, of the amino acid sequence of the invention present in said compound.

According to another preferred, but non-limiting aspect of the invention, the amino acid sequences of the invention are such that they bind to or otherwise associate with a human serum protein (such as human serum albumin) in such a way that, when the amino acid sequences are bound to or otherwise associated with said serum protein, the amino acid sequences exhibit a serum half-life in human of at least about 9 days (such as about 9 to 14 days), preferably at least about 10 days (such as about 10 to 15 days) or at least 11 days (such as about 11 to 16 days), more preferably at least about 12 days (such as about 12 to 18 days or more) or more than 14 days (such as about 14 to 19 days). Such amino acid sequences of the invention preferably can bind to said human serum protein (such as human serum albumin) with a dissociation constant (K_{D}) and/or with a binding affinity (K_{A}) that is as defined herein.

The invention also relates to compounds of the invention that comprise such an amino acid sequence and that have a half-life in human that is at least 80%, more preferably at least 90%, such as 95% or more or essentially the same as the half-life in human of the amino acid sequence present in said compound.

In one specific but non-limiting aspect, the amino acid sequences of the invention may be such that they are cross-reactive with the corresponding (i.e. orthologous) serum protein (such as serum albumin) from at least one further species of primate, and in particular with the corresponding (i.e. orthologous) serum protein (such as serum albumin) from at least one species of primate that is chosen from the group consisting of monkeys from the genus *Macaca* (such as rhesus monkeys or cynomologus monkeys) and baboons. Preferably, such cross-reactive amino acid sequences exhibit a serum half-life in said primate of at least about 50% (such as about 50% to 70%), preferably at least 60% (such as about 60% to 80%) or preferably at least 70% (such as about 70% to 90%), more preferably at least about 80% (such as about 80% to 90%) or preferably at least about 90% of the natural half-life of the corresponding (i.e. orthologous) serum protein (such as serum albumin) in said primate. Such amino acid sequences of the invention also preferably bind to the corresponding (i.e. orthologous) serum protein (such as serum albumin) from said primate with a dissociation constant (K_{D}) and/or with a binding affinity (K_{A}) that is as defined herein.

The invention also relates to compounds of the invention that comprise such an amino acid sequence and that have a half-life in human and/or in said at least one species of primate that is at least 80%, more preferably at least 90%, such as 95% or more or essentially the same as the half-life in human and/or said species of primate, respectively, of the amino acid sequence present in said compound.

In another specific, but non-limiting aspect, the amino acid sequences of the invention may be such that they bind to or otherwise associate with the corresponding (i.e. orthologous) serum protein (such as serum albumin) from at least one species of primate and that, when the half-life of the corresponding (i.e. orthologous) serum protein in the primate is at least about 10 days, such as between 10 and 15 days, for example about 11 to 13 days (by means of example, in rhesus monkeys, the expected half-life of serum albumin is between about 11 and 13 days, in particular about 11 to 12 days), have a serum half-life in said primate of least about 5 days (such as about 5 to 9 days), preferably at least about 6 days (such as about 6 to 10 days) or at least 7 days (such as about 7 to 11 days), more preferably at least about 8 days (such as about 8 to 12 days) or more than 9 days (such about 9 to 12 days or more). Such amino acid sequences of the invention are preferably further such that they bind to serum albumin from said species of primate with a dissociation constant (K_{D}) and/or with a binding affinity (K_{A}) that is as defined herein. In one specifically preferred aspect, such amino acid sequences are cross-reactive with human serum albumin, and more preferably bind to the corresponding (i.e. orthologous) serum protein (such as serum albumin) with a dissociation constant (K_{D}) and/or with a binding affinity (K_{A}) that is as defined herein.

The invention also relates to compounds of the invention that comprise such an amino acid sequence and that have a half-life in said at least one species of primate that is at least 80%, more preferably at least 90%, such as 95% or more or essentially the same as the half-life in said species of primate of the amino acid sequence present in said compound.

In another specific, but non-limiting aspect, the amino acid sequences of the invention may further be such that they bind to or otherwise associate the corresponding (i.e. orthologous) serum protein (such as serum albumin) from at least one species of primate and that, when the half-life of the corresponding (i.e. orthologous) serum protein (such as serum albumin) in the primate is at least about 13 days, such as between 13 and 18 days (by means of example, in baboons, the half-life of serum albumin is at least about 13 days, and usually about 16-18 days), have a serum half-life in said primate of least about 7 days (such as about 7 to 13 days), preferably at least about 8 days (such as about 8 to 15 days) or at least 9 days (such as about 9 to 16 days), more preferably at least about 10 days (such as about 10 to 16 days or more) or more than 13 days (such as about 13 to 18 days). Such amino acid sequences of the invention are preferably further such that they bind to the corresponding (i.e. orthologous) serum protein (such as serum albumin) from said species of primate with a dissociation constant (K_{D}) and/or with a binding affinity (K_{A}) that is as defined herein.

The invention also relates to compounds of the invention that comprise such an amino acid sequence and that have a half-life in said at least one species of primate that is at least 80%, more preferably at least 90%, such as 95% or more or essentially the same as the half-life in said species of primate of the amino acid sequence present in said compound.

In another specific, but non-limiting aspect, the amino acid sequences of the invention may be such that they:
a) bind to or otherwise associate with a human serum protein (such as serum albumin) in such a way that, when the amino acid sequences are bound to or otherwise associated with said human serum protein, the amino acid sequences exhibit a serum half-life in human of at least about 9 days (such as about 9 to 14 days), preferably at least about 10 days (such as about 10 to 15 days) or at least 11 days (such as about 11 to 16 days), more preferably at least about 12 days (such as about 12 to 18 days or more) or more than 14 days (such as about 14 to 19 days); and
b) are cross-reactive with the corresponding (i.e. orthologous) serum protein (such as serum albumin) from at least one primate chosen from species of the genus *Macaca* (and in particular with the corresponding (i.e. orthologous) serum protein from cynomologus monkeys and/or from rhesus monkeys): and
c) have a serum half-life in said primate of at least about 5 days (such as about 5 to 9 days), preferably at least about 6 days (such as about 6 to 10 days) or at least 7 days (such as about 7 to 11 days), more preferably at least about 8 days (such as about 8 to 12 days) or more than 9 days (such about 9 to 12 days or more).

Preferably, such amino acid sequences bind to the human protein (such as human serum albumin) and/or to the corresponding (i.e. orthologous) serum protein (such as serum albumin) from said species of primate with a dissociation constant (K_{D}) and/or with a binding affinity (K_{A}) that is as defined herein.

The invention also relates to compounds of the invention that comprise such an amino acid sequence and that have a half-life in human and/or in said at least one species of primate that is at least 80%, more preferably at least 90%, such as 95% or more or essentially the same as the half-life in human and/or said species of primate, respectively, of the amino acid sequence present in said compound:

In another specific, but non-limiting aspect, the amino acid sequences of the invention may be such that they:
a) bind to or otherwise associate with a human serum protein (such as serum albumin) in such a way that, when the amino acid sequences are bound to or otherwise associated with said human serum protein, the amino acid sequences exhibit a serum half-life in human of at least about 9 days (such as about 9 to 14 days), preferably at least about 10 days (such as about 10 to 15 days) or at least 11 days (such as about 11 to 16 days), more preferably at least about 12 days (such as about 12 to 18 days or more) or more than 14 days (such as about 14 to 19 days); and
b) are cross-reactive with the corresponding (i.e. orthologous) serum protein (such as serum albumin) from baboons; and
c) have a scrum half-life in baboons of least about 7 days (such as about 7 to 13 days), preferably at least about 8 days (such as about 8 to 15 days) or at least 9 days (such as about 9 to 16 days), more preferably at least about 10 days (such as about 10 to 16 days or more) or more than 13 days (such as about 13 to 18 days).

Preferably, such amino acid sequences bind to the human serum protein (such as human serum albumin) and/or to the corresponding (i.e. orthologous) serum protein (such as serum albumin) from baboon with a dissociation constant (K_{D}) and/or with a binding affinity (K_{A}) that is as defined herein.

The invention also relates to compounds of the invention that comprise such an amino acid sequence and that have a half-life in human and/or in said at least one species of primate that is at least 80%, more preferably at least 90%, such as 95% or more or essentially the same as the half-life in human and/or said species of primate, respectively, of the amino acid sequence present in said compound.

Preferably, also, the half-life of the compounds, constructs, fusion proteins, etc. comprising at least one amino acid sequence of the invention is preferably at least 80%, more preferably at least 90%, such as 95% or more or essentially the same as the half-life of the amino acid sequence of the invention present therein (i.e. in the same primate).

In a particular, but non-limiting aspect of the invention, the amino acid sequences of the invention (or compounds comprising the same) are directed against a serum protein that binds or can bind to the FcRn receptor (e.g. as part of recycling of said serum protein) and are such that they can bind to or otherwise associate with said serum protein in such a way that, when the amino acid sequence or polypeptide construct is bound to or otherwise associated with a said serum protein molecule, the binding of said serum protein molecule to FcRn is not (significantly) reduced or inhibited. Some specific, but non-limiting serum proteins that can bind to FcRn include serum albumin and immunoglobulins, such as in particular IgG.

In a further embodiment, the amino acid sequence of the invention (or compound comprising the same) can bind to or otherwise associate with a serum protein (such as serum albumin) in such a way that, when the amino acid sequence or polypeptide construct is bound to or otherwise associated with said serum protein molecule, the half-life of the serum protein molecule is not (significantly) reduced.

In a further embodiment the amino acid sequence of the invention (or compound comprising the same) is capable of binding to amino acid residues on the serum protein that are not involved in binding of said serum protein to FcRn. For example, when the serum protein is serum albumin, the amino acid sequence of the invention (or compound comprising the same) is capable of binding to amino acid residues that do not form pan of domain III of serum albumin.

In the invention, the amino acid sequence is an immunoglobulin variable domain sequence or a fragment thereof, e.g. a VH-, VL- or VHH-sequence or a fragment thereof. The amino acid sequence of the invention may be a domain antibody, "dAb", single domain antibody or Nanobody, or a fragment of any one thereof. The amino acid sequence of the invention may be a fully human, humanized, camelid, camelized human or humanized camelid sequence, and more specifically, may comprise 4 framework regions (FR1 to FR4 respectively) and 3 complementarity determining regions (CDR1 to CDR3 respectively).

More specifically, the amino acid sequence according to the invention is a (single) domain antibody or a Nanobody.

In other aspects, the invention relates to methods for generating the amino acid sequences of the invention, and in particular for generating the amino acid sequences of the invention that are directed against the desired or intended serum protein (such as human serum albumin). In one aspect, said method at least comprises the steps of:
a) providing a set, collection or library of amino acid sequences;
b) screening said set, collection or library of amino acid sequences for amino acid sequences that bind to the desired or intended serum protein at at least one physiological pH value more than 7.0, such as a pH value in the range of 7.2 to 7.4; and
c) screening said set, collection or library of amino acid sequences for amino acid sequences that bind to the desired or intended serum protein at at least one physiological pH value of less than 6.7, such as a pH value in the range of 6.5 to 5.5, with an association constant (K_{A}) that is at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.
   and
d) isolating the amino acid sequence(s) that bind to the desired or intended serum protein at at least one physiological pH value of less than 6.7, such as a pH in the range of 6.5 to 5.5, with an association constant (K_{A}) that is at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.

In particular, such a method can comprise the steps of:
a) providing a set, collection or library of amino acid sequences that are directed against the intended or desired serum protein; and
b) screening said set, collection or library of amino acid sequences for amino acid sequences that bind to the desired or intended serum protein at at least one physiological pH value more than 7.0, such as a pH value in the range of 7.2 to 7.4; and
c) screening said set, collection or library of amino acid sequences for amino acid sequences that bind to the desired or intended serum protein at at least one physiological pH value of less than 6.7, such as a pH value in the range of 6.5 to 5.5. with an association constant (K_{A}) that is at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH in the range of 7.2 to 7.4;
   and
d) isolating the amino acid sequence(s) that bind to the desired or intended serum protein at at least one physiological pH value of less than 6.7, such as a pH in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.

More in particular, such a method can comprise the steps of:
a) providing a set, collection or library of amino acid sequences that are directed against the intended or desired serum protein;
b) screening said set, collection or library of amino acid sequences for amino acid sequences that bind to the desired or intended serum protein at at least one physiological pH value more than 7.0, such as a pH value in the range of 7.2 to 7.4; and
c) screening said set, collection or library of amino acid sequences for amino acid sequences that bind to the desired or intended serum protein at at least one physiological pH value of less than 6.7, such as a pH value in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH in the range of 7.2 to 7.4;
   and
d) isolating the amino acid sequence(s) that bind to the desired or intended serum protein at at least one physiological pH value of less than 6.7, such as a pH in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.

Generally, in these methods, the step b) of screening the set, collection or library of amino acid sequences for amino acid sequences that bind to the desired or intended serum protein at at least one physiological pH value more than 7.0 (such as a pH value in the range of 7.2 to 7.4), is performed by screening at a physiological pH value more than 7.0 (such as a pH value in the range of 7.2 to 7.4). Similarly, step c) of screening the set, collection or library of amino acid sequences for amino acid sequences that bind to the desired or intended serum protein at at least one physiological pH value less than 6.7 (such as a pH value in the range of 6.5 to 5.5), is performed by screening at a physiological pH value less than 6.7 (such as a pH value in the range of 6.5 to 5.5).

As will be clear to the skilled person, the screening step can also be performed as a selection step. For example, antibody-antigen interactions are known to be often sensitive to changes in buffer conditions; pH and ionic strength, but most often those changes are not scored or investigated, and they are not often used to design drug therapeutics as variations are overall unpredictable. Binding proteins with the desirable binding characteristics are found for example by screening repertoires of binding proteins for the occurrence of a sensitive interaction, e.g. by carrying out a binding assay with two representative conditions (e.g. at pH 7.4 and at pH 6.0), and the relative binding strength determined. Such strength of relative interaction can be measured with any suitable binding test including ELISA, BIAcore-based methods, Scatchard analysis etc. Such test will reveal which binding proteins display interactions that are sensitive to the chosen parameter (pH) and to what extend. Binding proteins with the desirable binding characteristics are alternatively found by selecting repertoires of binding proteins, e.g. from phage, ribosome, yeast or cellular libraries using conditions in the selection that will preferentially enrich for the desirable sensitivity. Incubating a phage antibody library at basic pH (e.g. pH 7.4) and washing extensively the bound phage particles with a buffer of lower pH (e.g. 6.0) followed by acid elution (pH 2.0), will enrich for those phage antibodies that are recognizing antigen essentially independent of the pH. Binding proteins with the desirable binding characteristics can further be isolated from designer protein libraries in which the putative binding site has been engineered to not contain amino acid residues or sequences that are preferred in certain 'sensitive' interactions, e.g. histidines for pH-sensitivity. For example, it is known that the interaction between FcRn and IgG is exquisitely sensitive to pH, being reduced over 2 orders of magnitude as the pH is raised from pH 6.0 to 7.0. The main mechanistic basis of the affinity transition is the histidine content of the binding site : the imidazole side changes of histidine residues usually deprotonate over the pH range 6.0-7.0. The explicit exclusion of histidines in the putative binding site (e.g. using oligonucleotides that preferentially avoid this residue in the library, as with the use of trinucleotides and known in the field, e.g. Knappik et al, J. Mol. Biol 2000, vol 296:57-86) is predicted to yield a higher frequency of amino acid sequences that bind essentially independent of the pH.

Accordingly the term "screening" as used in the present description can comprise selection, screening or any suitable combination of selection and/or screening techniques.

In general, steps b) and c) can he performed as single or separate screening steps, or as part of a single screening process. When steps b) and c) are performed as part of a single screening process, such a screening process may for example comprise the steps of:
i) bringing the set, collection or library of amino acid sequences in contact with the serum protein at least one physiological pH value more than 7.0, such as a pH value in the range of 7.2 to 7.4;
ii) removing the amino acid sequence that do not bind in step i) (i.e. those amino acid sequences that do not bind to the serum protein with the desired association constant, as described herein); so that a set or collection of amino acid sequences remains that is bound to the serum protein;
iii) subjecting the set or collection of amino acid sequences to at least one physiological pH value of less than 6.7, such as a pH in the range of 6.5 to 5.5, such that amino acid sequences that bind to the serum protein at said pH value with the desired association constant stay bound to the serum protein, and such that amino acid sequences that do not bind to the serum protein at said pH value with the desired association constant stay do not stay bound to the serum protein;
iv) removing the amino acid sequence that do not bind in step iii) (i.e. those amino acid sequences that do not bind to the serum protein at said pH value with the desired association constant, as described herein);
   and optionally
v) collecting the amino acid sequences that in step iii) stay bound to the serum protein.

The set, collection or library of amino acid sequences used in the above method(s) can be any suitable set, collection or library of amino acid sequences. For example, the set, collection or library of amino acid sequences may be a set, collection or library of immunoglobulin sequences or of fragments of immunoglobulin sequences, such as a set, collection or library of immunoglobulin variable domain sequences or a fragments thereof, e.g. a set, collection or library of V_{H}-, V_{L}- or V_{HH}-sequences or a fragments thereof. In one specific, but non-limiting aspect, the set, collection or library of amino acid sequences a set, collection or library of domain antibodies, of proteins that can be used as domain antibodies, of "dAb", of single domain antibodies, of proteins that can be used as single domain antibodies, or of Nanobodies (or of suitable fragments of any of the foregoing).

The set, collection or library of amino acid sequences may be a naïve set, collection or library; may be a set, collection or library of synthetic or semi-synthetic amino acid sequences (for example, without limitation, a set, collection or library of amino acid sequences that has been generated by affinity maturation), or may be an immune set, collection or library. In one embodiment, the set, collection or library is an immune set, collection or library that has been obtained by suitably immunizing a mammal (such as a rabbit, rat, mouse, pig or dog, or Camelid) with an antigen (such that said mammal forms antibodies against said antigen), and then generating a set, collection or library of immunoglobulin sequences starting from a biological sample (such as blood or a sample of B-cells) obtained from said mammal. Methods and techniques for obtaining and screening such an immune set, collection or library will be clear to the skilled person, for example from the prior art cited herein. In one preferred aspect, the set, collection or library of immunoglobulin sequences is obtained from a mammal that has been suitably immunized with the intended serum protein (e.g. with serum albumin). In another preferred aspect, the set, collection or library is a set, collection or library of V_{HH} sequences obtained from a Camelid, and in particular an immune set, collection or library of V_{HH} sequences obtained from a Camelid that has been suitably immunized with the intended serum protein (e.g. with serum albumin).

The set, collection or library may contain any suitable number of amino acid sequences, such as 1, 2, 3 or about 5, 10, 50, 100, 500, 1000, 5000, 10⁴, 10⁵, 10⁶, 10⁷, 10⁸ or more sequences.

The above set, collection or library of amino acid sequences may contain one or more sequences that are not known in advance of the selection and/or screening process, for example if these sequences are the result of a randomization step (e.g. via error-prone PCR or other means) of one or more given amino acid sequences. Also, one or more or all of the amino acid sequences in the above set, collection or library of amino acid sequences may be obtained or defined by rational, or semi-empirical approaches such as computer modelling techniques or biostatics or data-mining techniques wherein amino acid sequences may have been defined or proposed that are predicted or expected to be endowed with certain properties such as increased stability, pH optimum, protease sensitivity or other properties or combinations thereof.

In such a set, collection or library (and/or during the screening steps described herein), the amino acid sequences present in said set, collection or library may also be suitably displayed on a suitable host or host cell, for example on phage particles, ribosomes, bacteria, yeast cells, etc. Again, suitable hosts or host cells, suitable techniques for displaying amino acid sequences on such hosts or host cells, and suitable techniques for screening a set, collection or library of amino acid sequences displayed on such hosts or host cells will be clear to the skilled person, for example from the prior art cited herein. When the amino acid sequence(s) are displayed on a suitable host or host cell, it is also possible (and customary) to first isolate from said host or host cell a nucleotide sequence that encodes the desired amino acid sequence, and then to obtain the desired amino acid sequence by suitably expressing said nucleotide sequence in a suitable host organism. Again, this can be performed in any suitable manner known per se, as will be clear to the skilled person.

By means of non-limiting example, such set, collection or library can comprise one, two or more amino acid sequences that are variants from one another (e.g. with designed point mutations or with randomized positions), compromise multiple amino acid sequences derived from a diverse set of naturally diversified amino acid sequences (e.g. an immune library), or any other source of diverse amino acid sequences (as described for example in Hoogenboom et al, Nat Biotechnol 23:1105, 2005 and Binz et al, Nat Biotechnol 2005, 23:1247). Such set, collection or library of amino acid sequences can be displayed on the surface of a phage particle, a ribosome, a bacterium, a yeast cell, a mammalian cell, and linked to the nucleotide sequence encoding the amino acid sequence within these carriers. This makes such set, collection or library amenable to selection procedures to isolate the desired amino acid sequences of the invention.

The method for generating the amino acid sequences of the invention can also comprise the steps of:
a) providing a set; collection or library of amino acid sequences for amino acid sequences that bind to the desired or intended serum protein at at least one physiological pH value more than 7.0, such as a pH value in the range of 7.2 to 7.4; and
b) screening said set, collection or library of amino acid sequences for amino acid sequences that bind to the desired or intended serum protein at at least one physiological pH value of less than 6.7, such as a pH value in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH in the range of 7.2 to 7.4;
   and
c) isolating the amino acid sequence(s) that bind to the desired or intended serum protein at at least one physiological pH value of less than 6.7, such as a pH in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.

In particular, such a method can comprise the steps of:
a) providing a set, collection or library of amino acid sequences for amino acid sequences that bind to the desired or intended serum protein at at least one physiological pH value more than 7.0, such as a pH value in the range of 7.2 to 7.4, with an association constant (K_{A}) of 10⁵ to 10¹² liter/ moles or more, and preferably 10⁷ to 10¹² liter/moles or more and more preferably 10⁸ to 10¹² liter/moles;
b) screening said set, collection or library of amino acid sequences for amino acid sequences that bind to the desired or intended serum protein at at least one physiological pH value of less than 6.7, such as a pH value in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH in the range of 7.2 to 7.4;
   and
c) isolating the amino acid sequence(s) that bind to the desired or intended serum protein at at least one physiological pH value of less than 6.7, such as a pH in the range of 6.5 to 5.5, with an association constant (K_{A}) that is at least 5%, such as at least 10%, preferably at least 25%, more preferably at least 50%, even more preferably at least 60%, such as even more preferably at least 70%, such as at least 80% or 90% or more (or even more than 100%, such as more than 110%, more than 120% or even 130% or more) of the association constant (K_{A}) of the amino acid sequence with respect to the same serum protein at at least one physiological pH value of more than 7.0, such as a pH value in the range of 7.2 to 7.4.

The method for generating the amino acid sequences of the invention can also comprise the steps of:
a) providing a set, collection or library of amino acid sequences for amino acid sequences that bind to the desired or intended serum protein at at least one physiological pH value in the range of 6.5 to 7.5 such as e.g. pH 7 or in the range of 7.2 to 7.4; and
b) screening said set, collection or library of amino acid sequences for amino acid sequences that interact with the desired or intended serum protein at at least one physiological pH value of between 5 to 6 such as e.g. pH 5.5 or in the range of 5.3 to 5.7, with an off-rate that is between 5 to 200%, such as 10 to 190%, e.g. 50 to 150%, e.g. 70 to 130% of the off-rate of the amino acid sequence with respect to the same serum protein at at least one physiological pH value of between 5 to 6 such as e.g. pH 5.5 or in the range of 5.3 to 5.7; and
c) isolating said amino acid sequence(s) with the above off-rate profile; and optionally
d) evaluating the half life of said amino acid sequence in vivo.

The method for generating the amino acid sequences of the invention wherein the off-rates of said amino acid sequences in the different pH conditions is within 50 and 150% (more preferably 80 to 120%), i.e. substantially independent of the pH.

In one embodiment, the invention relates to a compound comprising at least one amino acid sequence of the invention (also referred to herein as a "*compound of the invention*"), which compound may optionally further comprise at least one therapeutic moiety, comprising therapeutic moieties selected from at least one of the group consisting of small molecules, polynucleotides, polypeptides or peptides. The compound of the invention is suitable for administration to a primate with a frequency corresponding to not less than 50% (such as about 50% to 70%), preferably at least 60% (such as about 60% to 80%) or preferably at least 70% (such as about 70% to 90%), more preferably at least about 80% (such as about 80% to 90%) or preferably at least about 90% of the natural half-life of the serum protein (such as serum albumin) in said primate, or, alternatively, at intervals of at least 4 days (such as about 4 to 12 days or more), preferably at least 7 days (such as about 7 to 15 days or more), more preferably at least 9 days (such as about 9 to 17 days or more), such as at least 15 days (such as about 15 to 19 days or more, in particular for administration to man) or at least 17 days (such as about 17 to 19 days or more, in particular for administration to man); where such administrations are in particular made to maintain the desired level of the compound in the serum of the subject that is treated with the compound (such *inter alia* dependent on the compound used and/or the disease to be treated, as will be clear to the skilled person. The clinician or physician will be able to select the desired serum level and to select the dose(s) and/or amount(s) to be administered to the subject to be treated in order to achieve and/or to maintain the desired serum level in said subject, when the compound of the invention is administered at the frequencies mentioned herein. For example, such a dose can range between 1 times and 10 times the desired serum level, such as between 2 times and 4 times the desired serum level (in which the desired serum level is recalculated in a manner known per se so as to provide a corresponding dose to be administered).

The compounds of the invention may also be formulated as unit doses that are intended and/or packaged (e.g. with suitable instructions for use) for administration at the aforementioned frequencies, and such unit doses and packaged products form further aspects of the invention. Another aspect of the invention relates to the use of a compound of the invention in providing such a unit dose or packaged product (i.e. by suitably formulating and/or packaging said compound).

In a particular embodiment, the compound of the invention is a fusion protein or construct. In said fusion protein or construct the amino acid sequence of the invention may be either directly linked to the at least one therapeutic moiety or is linked to the at least one therapeutic moiety via a linker or spacer. A particular embodiment relates to a therapeutic moiety comprising an immunoglobulin sequence or a fragment thereof, more specifically a (single) domain antibody or a Nanobody.

The invention also relates to multivalent and multispecific Nanobody constructs, comprising at least one amino acid sequence of the invention which is a Nanobody and at least one further Nanobody. The Nanobody is either directly linked to the at least one further Nanobody or is linked to the at least one further Nanobody via a linker or spacer, preferably linked to the at least one further Nanobody via an amino acid sequence linker or spacer.

Furthermore, the invention relates to nucleotide sequence or nucleic acid that encode an amino acid sequence according to the invention, or the amino acid sequence of a compound according to the invention, or the multivalent and multispecific Nanobody of the invention. The invention also provides hosts or host cells that contain a nucleotide sequence or nucleic acid of the invention and/or that express (or are capable of expressing) an amino acid sequence of the invention, or the amino acid sequence of a compound according to the invention, or the multivalent and multispecific Nanobody of the invention.

Moreover, the invention relates to method for preparing an amino acid sequence, compound, or multivalent and multispecific Nanobody of the invention comprising cultivating or maintaining a host cell of the invention under conditions such that said host cell produces or expresses the said product, and optionally further comprises the said product so produced.

In one embodiment, the invention relates to a pharmaceutical composition comprising one or more selected from the group consisting of the amino acid sequence, compound, or multivalent and multispecific Nanobody of the invention, wherein said pharmaceutical composition is suitable for administration to a primate at intervals of at least about 50% of the natural half-life of the serum protein in said primate. The pharmaceutical composition may further comprise at least one pharmaceutically acceptable carrier, diluent or excipient.

The invention also encompasses medical uses and methods of treatment encompassing the amino acid sequence, compound or multivalent and multispecific Nanobody of the invention, wherein said medical use or method is characterized in that said medicament is suitable for administration at intervals of at least about 50% of the natural half-life of the serum protein in said primate, and the method comprises administration at a frequency of at least about 50% of the natural half-life of the serum protein in said primate.

The invention also relates to methods for extending or increasing the serum half-life of a therapeutic. The methods include contacting the therapeutic with any of the foregoing amino acid sequences, compounds, fusion proteins or constructs of the invention (including multivalent and multispecific Nanobodies), such that the therapeutic is bound to or otherwise associated with the amino acid sequences, compounds, fusion proteins or constructs of the invention. In some embodiments, the therapeutic is a biological therapeutic, preferably a peptide or polypeptide, in which case the step of contacting the therapeutic can include preparing a fusion protein by linking the peptide or polypeptide with the amino acid sequence, compound, fusion proteins or constructs of the invention.

These methods can further include administering the therapeutic to a primate after the therapeutic is bound to or otherwise associated with the amino acid sequence, compound, fusion protein or construct of the invention. In such methods, the serum half-life of the therapeutic in the primate is at least 1.5 times the half-life of therapeutic per se, or is increased by at least 1 hour compared to the half-life of therapeutic per se. In some preferred embodiments, the serum half-life of the therapeutic in the primate is at least 2 times, at least 5 times, at least 10 times or more than 20 times greater than the half-life of the corresponding therapeutic moiety per se. In other preferred embodiments, the serum half-life of the therapeutic in the primate is increased by more than 2 hours, more than 6 hours or more than 12 hours compared to the half-life of the corresponding therapeutic moiety per se.

Preferably, the serum half-life of the therapeutic in the primate is increased so that the therapeutic has a half-life that is as defined herein for the compounds of the invention (i.e. in human and/or in at least one species of primate).

In another aspect, the invention relates to a method for modifying a therapeutic such that the desired therapeutic level of said therapeutic is, upon suitable administration of said therapeutic so as to achieve said desired therapeutic level, maintained for a prolonged period of time.

The methods include contacting the therapeutic with any of the foregoing amino acid sequences, compounds, fusion proteins or constructs of the invention (including multivalent and multispecific Nanobodies), such that the therapeutic is bound to or otherwise associated with the amino acid sequences, compounds, fusion proteins or constructs of the invention. In some embodiments, the therapeutic is a biological therapeutic, preferably a peptide or polypeptide, in which case the step of contacting the therapeutic can include preparing a fusion protein by linking the peptide or polypeptide with the amino acid sequence, compound, fusion proteins or constructs of the invention.

These methods can further include administering the therapeutic to a primate after the therapeutic is bound to or otherwise associated with the amino acid sequence, compound, fusion protein or construct of the invention, such that the desired therapeutic level is achieved upon such administration. In such methods, the time that the desired therapeutic level of said therapeutic is maintained upon such administration is at least 1.5 times the half-life of therapeutic per se, or is increased by at least 1 hour compared to the half-life of therapeutic per se. In some preferred embodiments, the time that the desired therapeutic level of said therapeutic is maintained upon such administration is at least 2 times, at least 5 times, at least 10 times or more than 20 times greater than the half-life of the corresponding therapeutic moiety per se. In other preferred embodiments, the time that the desired therapeutic level of said therapeutic is maintained upon such administration is increased by more than 2 hours, more than 6 hours or more than 12 hours compared to the half-life of the corresponding therapeutic moiety per se.

Preferably, the time that the desired therapeutic level of said therapeutic is maintained upon such administration is increased such that the therapeutic can be administered at a frequency that is as defined herein for the compounds of the invention.

In another aspect, the invention relates to the use of a compound of the invention (as defined herein) for the production of a medicament that increases and/or extends the level of the therapeutic agent in said compound or construct in the serum of a patient such that said therapeutic agent in said compound or construct is capable of being administered at a lower dose as compared to the therapeutic agent alone (i.e. at essentially the same frequency of administration).

### Detailed description of the invention

In one aspect, the invention achieves this objective by providing immunoglobulin variable domain sequences, that, at physiological values of the pH, hind to serum proteins in a manner that is essentially independent of the pH (as defined herein).

Said amino acid sequences are also preferably such that they can bind to or otherwise associate with the serum protein (such as serum albumin) in such a way that, when the amino acid sequence or polypeptide construct is bound to or otherwise associated with the serum protein molecule in a primate, they exhibit a serum half-life of at least about 50% of the natural half-life of the serum protein in said primate, preferably at least about 60%, preferably at least about 70%, more preferably at least about 80% and most preferably at least about 90%.

The serum half-life of the amino acid sequences of the invention after administration to a primate may be at least 50%, 55%, 60%, 65%, 70%, 75%, 80%, 85%, 90%, 95% or at least 100% of the natural half-life of the serum protein in said primate.

By "natural serum half-life of the serum protein in said primate" is meant the serum half-life as defined below, which the serum protein has in healthy individuals under physiological conditions. Taking serum albumin as an example of the serum protein, the natural serum half-life of serum albumin in humans is 19 days. Smaller primates are known to have shorter natural half-lives of serum albumin, e.g. in the range of 8 to 19 days. Specific half-lives of serum albumin may be at least 4, 5, 6, 7, 8, 9, 10, 11, 12, 13, 14, 15, 16, 17, 18, or 19 days or more.

From this it follows, that for example in a human individual, an amino acid sequence of the invention shows a serum half-life in association with serum albumin of at least about 50% of 19 days, i.e. 7.6 days. In smaller primates, the serum half-life may be shorter in days, depending on the natural half-lives of serum albumin in these species.

In the present description, the term "primate" refers to both species of monkeys an apes, and includes species of monkeys such as monkeys from the genus *Macaca.* (such as, and in particular, cynomologus monkeys (*Macaca fascicularis*) and/or rhesus monkeys (*Macaca mulatta*)) and baboon (*Papio ursinus*)), as well as marmosets (species from the genus *Callithrix*), squirrel monkeys (species from the genus *Saimiri*) and tamarins (species from the genus *Saguinus*), as well as species of apes such as chimpanzees (*Pan troglodytes*), and also includes man. Humans are the preferred primate according to the invention.

The half-life of an amino acid sequence or compound can generally be defined as the time taken for the serum concentration of the polypeptide to be reduced by 50%, in vivo, for example due to degradation of the sequence or compound and/or clearance or sequestration of the sequence or compound by natural mechanisms. The half-life of the amino acid sequences of the invention (and of compounds comprising the same) in the relevant species of primate can be determined in any manner known per se, such as by pharmacokinetic analysis. Suitable techniques will be clear to the person skilled in the art, and may for example generally involve the steps of suitably administering to the primate a suitable dose of the amino acid sequence or compound to be treated; collecting blood samples or other samples from said primate at regular intervals; determining the level or concentration of the amino acid sequence or compound of the invention in said blood sample; and calculating, from (a plot of) the data thus obtained, the time until the level or concentration of the amino acid sequence or compound of the invention has been reduced by 50% compared to the initial level upon dosing. Reference is for example made to standard handbooks, such as Kenneth, A et al: Chemical Stability of Pharmaceuticals: A Handbook for Pharmacists and in Peters et al, Pharmacokinete analysis: A Practical Approach (1996). Reference is also made to "Pharmacokinetics", M Gibaldi & D Perron, published by Marcel Dekker, 2nd Rev. edition (1982).

As described on pages 6 and 7 of WO 04/003019 and in the further references cited therein, the half-life can be expressed using parameters such as the t1/2-alpha, t1/2-beta and the area under the curve (AUC). In the present specification, an "increase in half-life" refers to an increase in any one of these parameters, such as any two of these parameters, or essentially all three these parameters. An "increase in half-life" in particular refers to an increase in the tl/2-beta, either with or without an increase in the t1/2-alpha and/or the AUC or both.

In another aspect, the amino acid sequences of the invention, and in particular immunoglobulin sequences of the invention, and more in particular immunoglobulin variable domain sequences of the invention, directed against a serum protein (such as serum albumin, preferably human serum albumin), are such that they that have a half-life in rhesus monkeys of at least about 4, preferably at least about 7, more preferably at least about 9 days.

In yet another aspect, the amino acid sequences of the invention are such that they have a half-life in human of at least about 7, preferably at least about 15, more preferably at least about 17 days. The invention also relates to compounds of the invention that have a half-life in human that is at least 80%, more preferably at least 90%, such as 95% or more or essentially the same as the half-life of the amino acid sequence of the invention present in said compound. More in particular, the invention also relates to compounds of the invention that have a half-life in human of at least about 7, preferably at least about 15, more preferably at least about 17 days.

The invention also provides compounds comprising the amino acid sequence of the invention, in particular compounds comprising at least one therapeutic moiety in addition to the amino acid sequence of the invention. The compounds according to the invention are characterized by exhibiting a comparable serum half-life in primates to the amino acid sequence of the invention, more preferable a half-life which is at least the serum half-life of the amino acid sequence of the invention, and more preferably a half-life which is higher than the half-life of the amino acid sequence of the invention in primates.

In one aspect, the invention achieves this objective by providing the amino acid sequences disclosed herein, that can bind to a serum protein that can bind to FcRn, which amino acid sequences are further such that they can bind to or otherwise associate with the serum protein (such as serum albumin) in such a way that, when the amino acid sequence or polypeptide construct is bound to or otherwise associated with the serum protein molecule, the binding of said serum protein molecule to FcRn is not (significantly) reduced or inhibited (i.e. compared to the binding of said serum protein molecule to FcRn when the amino acid sequence or polypeptide construct is not bound thereto). In this aspect of the invention, by "not significantly reduced or inhibited" is meant that the binding affinity for serum protein to FcRn (as measured using a suitable assay, such as SPR) is not reduced by more than 50%, preferably not reduced by more than 30 %, even more preferably not reduced by more than 10%, such as not reduced by more than 5%, or essentially not reduced at all. In this aspect of the invention, "not significantly reduced or inhibited" may also mean (or additionally mean) that the half-life of the serum-protein molecule is not significantly reduced (as defined below).

When in this description, reference is made to binding, such binding is preferably specific binding, as normally understood by the skilled person.

When an amino acid sequence as described herein is a monovalent immunoglobulin sequence (for example, a monovalent Nanobody), said monovalent immunoglobulin sequence preferably binds to human serum albumin with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles, and/or with a binding affinity (K_{A}) of at least 10⁷ M⁻¹, preferably at least 10⁸ M⁻¹, more preferably at least 10⁹ M⁻¹, such as at least 10¹² M⁻¹. Any K_{D} value greater than 10⁴ mol/liter (or any K_{A} value lower than 10⁴ M⁻¹) liters/mol is generally considered to indicate non-specific binding. Preferably, a monovalent immunoglobulin sequence of the invention will bind to the desired serum protein with an affinity less than 500 nM, preferably less than 200 nM, more preferably less than 10 nM, such as less than 500 pM. Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art.

In another aspect, the immunoglobulin variable domain equences of the invention, are further such that they can bind to or otherwise associate with a serum protein (such as serum albumin) in such a way that, when the amino acid sequence or polypeptide construct is bound to or otherwise associated with said serum protein molecule, the half-life of said serum protein molecule is not (significantly) reduced (i.e. compared to the half-life of the serum protein molecule when the amino acid sequence or polypeptide construct is not bound thereto). In this aspect of the invention, by "not significantly reduced" is meant that the half-life of the serum protein molecule (as measured using a suitable technique known per se) is not reduced by more than 50%, preferably not reduced by more than 30%, even more preferably not reduced by more than 10%, such as not reduced by more than 5%, or essentially not reduced at all.

In another aspect, the immunoglobulin variable domain sequences of the invention may be directed against serum proteins that can bind to FcRn, and may be further such that they are capable of binding to amino acid residues on the serum protein molecule (such as amino acid residues on serum albumin) that are not involved in binding of said serum protein to FcRn. In particular, according to this aspect of the invention, when the amino acid sequences of the invention are directed against serum albumin, they are such that they are capable of binding to amino acid sequences of serum albumin that do not form part of domain III of serum albumin. For example, but without being limited thereto, this aspect of the invention provides amino acid sequences that are capable of binding to amino acid sequences of serum albumin that form part of domain I and/or domain II.

The amino acid sequences of the invention are preferably (single) domain antibodies or suitable for use as (single) domain antibodies, and as such may be heavy chain variable domain sequence (VH sequence) or a light chain variable domain sequence (VL sequence), and preferably are VH sequences. The amino acid sequences may for example be so-called "dAbs".

However, according to a particularly preferred embodiment, the amino acid sequences of the present invention are Nanobodies. For a further description and definition of Nanobodies, as well as of some of the further terms used in the present description (such as, for example and without limitation, the term "directed against") reference is made to the copending patent applications by Ablynx N.V. (such as WO 06/040153 and the copending International application WO-A-2006122786; as well as the further prior art cited therein.

As such, they may be Nanobodies belonging to the "KERE"-class. to the "GLEW"-class or to the "103-P,R,S"-class (again as defined in the copending patent applications by Ablynx N.V.).

Preferably, the amino acid sequences of the present invention are humanized Nanobodies (again as defined in the copending patent applications by Ablynx N.V.).

The amino acid sequences disclosed herein can be used with advantage as a fusion partner in order to increase the half-life of therapeutic moieties such as proteins, compounds (including, without limitation, small molecules) or other therapeutic entities.

Thus, in another aspect, the invention provides proteins or polypeptides that comprise or essentially consist of an amino acid sequence as disclosed herein. In particular, the invention provides protein or polypeptide constructs that comprise or essentially consist of at least one amino acid sequence of the invention that is linked to at least one therapeutic moiety, optionally via one or more suitable linkers or spacers. Such protein or polypeptide constructs may for example (without limitation) be a fusion protein. as further described herein.

The invention further relates to therapeutic uses of protein or polypeptide constructs or fusion proteins and constructs and to pharmaceutical compositions comprising such protein or polypeptide constructs or fusion proteins.

In some embodiments the at least one therapeutic moiety comprises or essentially consists of a therapeutic protein, polypeptide, compound, factor or other entity. In a preferred embodiment the therapeutic moiety is directed against a desired antigen or target, is capable of binding to a desired antigen (and in particular capable of specifically binding to a desired antigen), and/or is capable of interacting with a desired target. In another embodiment, the at least one therapeutic moiety comprises or essentially consists of a therapeutic protein or polypeptide. In a further embodiment, the at least one therapeutic moiety comprises or essentially consists of an immunoglobulin or immunoglobulin sequence (including but not limited to a fragment of an immunoglobulin), such as an antibody or an antibody fragment (including but not limited to an ScFv fragment). In yet another embodiment, the at least one therapeutic moiety comprises or essentially consists of an antibody variable domain, such as a heavy chain variable domain or a light chain variable domain.

In a preferred embodiment, the at least one therapeutic moiety comprises or essentially consists of at least one domain antibody or single domain antibody, "dAb" or Nanobody®. According to this embodiment, the amino acid sequence of the invention is preferably also a domain antibody or single domain antibody, "dAb" or Nanobody, so that the resulting construct or fusion protein is a multivalent construct (as described herein) and preferably a multispecific construct (also as defined herein) comprising at least two domain antibodies, single domain antibodies, "dAbs" or Nanobodies® (or a combination thereof), at least one of which is an amino acid sequence of the invention.

In a specific embodiment, the at least one therapeutic moiety comprises or essentially consists of at least one monovalent Nanobody® or a bivalent, multivalent, bispecific or multispecific Nanobody® construct. According to this embodiment, the amino acid sequence of the invention is preferably also a Nanobody, so that the resulting construct or fusion protein is a multivalent Nanobody construct (as described herein) and preferably a multispecific Nanobody construct (also as defined herein) comprising at least two Nanobodies, at least one of which is an amino acid sequence of the invention.

According to one embodiment of the invention, the amino acid sequence of the invention is a humanized Nanobody.

Also, when the amino acid sequences, proteins, polypeptides or constructs of the invention are intended for pharmaceutical or diagnostic use, the aforementioned are preferably directed against a human serum protein, such as human serum albumin.

When the amino acid sequence is an immunoglobulin sequence such as a immunoglobulin variable domain sequence, a suitable (i.e. suitable for the purposes mentioned herein) fragment of such a sequence may also be used. For example, when the amino acid sequence is a Nanobody, such a fragment may essentially be as described in WO 04/041865.

The invention also relates to a protein or polypeptide that comprises or essentially consists of an amino acid sequence as described herein, or a suitable fragment thereof.

The amino acid sequences of the invention may also contain one or more additions binding sites for one or more other antigens, antigenic determinants, proteins, polypeptides, or other compounds.

As mentioned herein, the amino acid sequences described herein can be used with advantage as a fusion partner in order to increase the half-life of therapeutic moieties such as proteins, compounds (including, without limitation, small molecules) or other therapeutic entities. Thus, one embodiment of the invention relates to a construct or fusion protein that comprises at least one amino acid sequence of the invention and at least one therapeutic moieties. Such a construct or fusion protein preferably has increased half-life, compared to the therapeutic moiety per se. Generally, such fusion proteins and constructs can be (prepared and used) as described in the prior art cited above, but with an amino acid sequence of the invention instead of the half-life increasing moieties described in the prior art.

Generally, the constructs or fusion proteins described herein preferably have a half-life that is at least 1.5 times, preferably at least 2 times, such as at least 5 times, for example at least 10 times or more than 20 times, greater than the half-life of the corresponding therapeutic moiety per se.

Also, preferably, any such fusion protein or construct has a half-life that is increased with more than 1 hour, preferably more than 2 hours, more preferably of more than 6 hours, such as of more than 12 hours, compared to the half-life of the corresponding therapeutic moiety per se.

Also, preferably, any fusion protein or construct has a half-life that is more than 1 hour, preferably more than 2 hours, more preferably of more than 6 hours, such as of more than 12 hours, and for example of about one day, two days, one week, two weeks or three weeks; and preferably no more than 2 months, although the latter may be less critical.

Also, as mentioned above, when the amino acid sequence of the invention is a Nanobody, it can be used to increase the half-life of other immunoglobulin sequences, such as domain antibodies, single domain antibodies, "dAbs" or Nanobodies.

Thus, one embodiment of the invention relates to a construct or fusion protein that comprises at least one amino acid sequence of the invention and at least one immunoglobulin sequence, such as a domain antibodies, single domain antibodies, "dAbs" or Nanobodies. The immunoglobulin sequence is preferably directed against a desired target (which is preferably a therapeutic target), and/or another immunoglobulin sequence that useful or suitable for therapeutic, prophylactic and/or diagnostic purposes.

Thus, in another aspect, the invention relates to a multispecific (and in particular bispecific) Nanobody constructs that comprises at least one Nanobody as described herein, and at least one other Nanobody, in which said at least one other Nanobody is preferably directed against a desired target (which is preferably a therapeutic target), and/or another Nanobody that useful or suitable for therapeutic, prophylactic and/or diagnostic purposes.

For a general description of Nanobodies and of multivalent and multispecific polypeptides containing one or more Nanobodies and their preparation, reference is made to the co-pending applications by Ablynx N.V. such as WO 06/040153 and the copending International application PCT/EP2006/004678 (as well as the further prior art cited in these applications), and also to for example Conrath et al., J. Biol. Chem., Vol. 276, 10. 7346-7350, 2001; Muyldermans, Reviews in Molecular Biotechnology 74 (2001), 277-302; as well as to for example WO 96/34103 and WO 99/23221. Some other examples of some specific multispecific and/or multivalent polypeptide of the invention can be found in the co-pending applications by Ablynx N.V.. In particular, for a general description of multivalent and multispecific constructs comprising at least one Nanobody against a serum protein for increasing the half-life, of nucleic acids encoding the same, of compositions comprising the same, of the preparation of the aforementioned, and of uses of the aforementioned, reference is made to the International application WO 04/041865 by Ablynx N.V.. The amino acid sequences described herein can generally be used analogously to the half-life increasing Nanobodies described therein.

In one non-limiting embodiment, said other Nanobody is directed against tumor necrosis factor alpha (TNF-alpha), in monomeric and/or multimeric (i.e. trimeric) form. Some examples of such Nanobody constructs can be found in the copending International application by Ablynx N.V. entitled "*Improved Nanobodies*™ *against Tumor Necrosis Factor-alpha*", which has the same priority and the same international filing date as the present application.

The invention also relates to nucleotide sequences or nucleic acids that encode amino acid sequences, compounds, fusion proteins and constructs described herein. The invention further includes genetic constructs that include the foregoing nucleotide sequences or nucleic acids and one or more elements for genetic constructs known per se. The genetic construct may be in the form of a plasmid or vector. Again, such constructs can be generally as described in the co-pending patent applications by Ablynx N.V. and prior art mentioned herein and in the further prior art cited therein.

The invention also relates to hosts or host cells that contain such nucleotide sequences or nucleic acids, and/or that express (or are capable of expressing), the amino acid sequences, compounds, fusion proteins and constructs described herein. Again, such host cells can be generally as described in the co-pending patent applications by Ablynx N.V. and prior art mentioned herein, and in the further prior art cited therein.

The invention also relates to a method for preparing an amino acid sequence, compound, fusion protein or construct as described herein, which method comprises cultivating or maintaining a host cell as described herein under conditions such that said host cell produces or expresses an amino acid sequence, compound, fusion protein or construct as described herein, and optionally further comprises isolating the amino acid sequence, compound, fusion protein or construct so produced. Again, such methods can be performed as generally described in the co-pending patent applications by Ablynx N.V. and prior art mentioned herein, and in the further prior art cited therein.

The invention also relates to a pharmaceutical composition that comprises at least one amino acid sequence, compound, fusion protein or construct as described herein, and optionally at least one pharmaceutically acceptable carrier, diluent or excipient Such preparations, carriers, excipients and diluents may generally be as described in the copending patent applications by Ablynx N.V. and prior art mentioned herein, and in the further prior art cited therein.

However, since the amino acid sequences, compounds, fusion proteins or constructs described herein have an increased half-life, they are preferably administered to the circulation. As such, they can be administered in any suitable manner that allows the amino acid sequences, compound, fusion proteins or constructs to enter the circulation, such as intravenously, via injection or infusion, or in any other suitable manner (including oral administration, administration through the skin, transmucosal administration, intranasal administration, administration via the lungs, etc) that allows the amino acid sequences, compounds, fusion proteins or constructs to enter the circulation. Suitable methods and routes of administration will be clear to the skilled person, again for example also from the teaching of WO 04/041862.

Thus, in another aspect, the invention relates to use in a method for the prevention and/or treatment of at least one disease or disorder that can be prevented or treated by the use of a compound, fusion protein or construct as described herein, which method comprises administering, to a subject in need thereof, a pharmaceutically active amount of an amino acid sequence, compound, fusion protein or construct of the invention, and/or of a pharmaceutical composition comprising the same. The diseases and disorders that can be prevented or treated by the use of an amino acid sequence, compound, fusion protein or construct as described herein will generally be the same as the diseases and disorders that can be prevented or treated by the use of the therapeutic moiety that is present in the amino acid sequence, compound, fusion protein or construct of the invention.

The subject to be treated may be any primate, but is in particular a human being. As will be clear to the skilled person, the subject to be treated will in particular be a person suffering from, or at risk from, the diseases and disorders mentioned herein.

More specifically, the present invention relates to use in a method of treatment wherein the frequency of administering the amino acid sequence, compound, fusion protein or construct of the invention is at least 50% of the natural half-life of the serum protein against which the amino acid sequence, compound, fusion protein or construct of the invention is directed. preferably at least 60%, preferably at least 70%, more preferably at least 80% and most preferably at least 90%.

Specific frequencies of administration to a primate, which are within the scope of the present invention are at least 50%, 55%, 60%, 65%, 70%, 75%, 80%, 85%, 90%, 95% or at least 100% of the natural half-life of the serum protein against which the amino acid sequence, compound, fusion protein or constructs of the invention is directed.

In other words, specific frequencies of administration which are within the scope of the present invention are every 4, 5, 6, 7, 8, 9, 10, 11, 12, 13, 14, 15, 16, 17, 18, or 19 days.

Without limitation, the frequencies of administration referred to above are in particular suited for maintaining a desired level of the amino acid sequence, compound, fusion protein or construct in the serum of the subject treated with the amino acid sequence, compound, fusion protein or construct, optionally after administration of one or more (initial) doses that are intended to establish said desired serum level. As will be clear to the skilled person, the desired serum level may *inter alia* be dependent on the amino acid sequence, compound, fusion protein or construct used and/or the disease to be treated. The clinician or physician will be able to select the desired serum level and to select the dose(s) and/or amount(s) to be administered to the subject to be treated in order to achieve and/or to maintain the desired serum level in said subject, when the amino acid sequence, compound, fusion protein or construct of the invention is administered at the frequencies mentioned herein.

In the context of the present invention, the term "prevention and/or treatment" not only comprises preventing and/or treating the disease, but also generally comprises preventing the onset of the disease, slowing or reversing the progress of disease, preventing or slowing the onset of one or more symptoms associated with the disease, reducing and/or alleviating one or more symptoms associated with the disease, reducing the severity and/or the duration of the disease and/or of any symptoms associated therewith and/or preventing a further increase in the severity of the disease and/or of any symptoms associated therewith, preventing, reducing or reversing any physiological damage caused by the disease, and generally any pharmacological action that is beneficial to the patient being treated.

The subject to be treated may be any primate, but is in particular a human being. As will be clear to the skilled person, the subject to be treated will in particular be a person suffering from, or at risk from, the diseases and disorders treatable by the therapeutic moiety mentioned herein.

In another embodiment, the invention relates to a use in method for immunotherapy, and in particular for passive immunotherapy, which method comprises administering, to a subject suffering from or at risk of the diseases and disorders mentioned herein, a pharmaceutically active amount of an amino acid sequence, compound, fusion protein or construct of the invention, and/or of a pharmaceutical composition comprising the same.

The invention also relates to methods for extending or increasing the serum half-life of a therapeutic. In these methods, the therapeutic is contacted with any of the amino acid sequences, compounds, fusion proteins or constructs of the invention, including multivalent and multispecific Nanobodies, such that the therapeutic is bound to or otherwise associated with the amino acid sequences, compounds, fusion proteins or constructs.

The therapeutic and the amino acid sequences, compounds, fusion proteins or constructs can be bound or otherwise associated in various ways known to the skilled person. In the case of biological therapeutics, such as a peptide or polypeptide, the therapeutic can be fused to the amino acid sequences, compounds, fusion proteins or constructs according to methods known in the art. The therapeutic can be directly fused, or fused using a spacer or linker molecule or sequence. The spacer or linker are, in preferred embodiment, made of amino acids, but other non-amino acid spacers or linkers can be used as is well known in the art Thus, the step of contacting the therapeutic can include preparing a fusion protein by linking the peptide or polypeptide with the amino acid sequences, compounds, fusion proteins or constructs of the invention, including multivalent and multispecific Nanobodies.

The therapeutic also can be bound directly by the amino acid sequences, compounds, fusion proteins or constructs of the invention. As one example, a multivalent and multispecific Nanobody, can include at least one variable domain that binds the serum protein (such as serum albumin) and at least one variable domain that binds the therapeutic.

The methods for extending or increasing serum half-life of a therapeutic can further include administering the therapeutic to a primate after the therapeutic is bound to or otherwise associated with the amino acid sequence, compound, fusion proteins or constructs of the invention. In such methods the half-life of the therapeutic is extended or increased by significant amounts, as is described elsewhere herein.

The amino acid sequence, compound, fusion protein or construct and/or the compositions comprising the same are administered according to a regime of treatment that is suitable for preventing and/or treating the disease or disorder to be prevented or treated. The clinician will generally be able to determine a suitable treatment regimen, depending on factors such as the disease or disorder to be prevented or treated, the seventy of the disease to be treated and/or the severity of the symptoms thereof, the specific Nanobody or polypeptide of the invention to be used, the specific route of administration and pharmaceutical formulation or composition to be used, the age, gender, weight, diet, general condition of the patient, and similar factors well known to the clinician.

Generally, the treatment regimen will comprise the administration of one or more amino acid sequences, compounds, fusion proteins or constructs of the invention, or of one or more compositions comprising the same, in one or more pharmaceutically effective amounts or doses. The specific amount(s) or doses to administered can be determined by the clinician, again based on the factors cited above.

Generally, for the prevention and/or treatment of the diseases and disorders mentioned herein and depending on the specific disease or disorder to be treated, the potency and/or the half-life of the specific amino acid sequences, compounds, fusion proteins or constructs to be used, the specific route of administration and the specific pharmaceutical formulation or composition used, the Nanobodies and polypeptides of the invention will generally be administered in an amount between 1 gram and 0.01 microgram per kg body weight per day, preferably between 0.1 gram and 0.1 microgram per kg body weight per day, such as about 1, 10, 100 or 1000 microgram per kg body weight per day, either continuously (e.g. by infusion, as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his expert judgment. Generally, some guidance on the amounts to be administered can be obtained from the amounts usually administered for comparable conventional antibodies or antibody fragments against the same target administered via essentially the same route, taking into account however differences in affinity/avidity, efficacy, biodistribution, half-life and similar factors well known to the skilled person.

Usually, in the above method, a single Nanobody or polypeptide of the invention will be used. It is however within the scope of the invention to use two or more Nanobodies and/or polypeptides of the invention in combination.

The Nanobodies and polypeptides of the invention may also be used in combination with one or more further pharmaceutically active compounds or principles, i.e. as a combined treatment regimen, which may or may not lead to a synergistic effect. Again, the clinician will be able to select such further compounds or principles, as well as a suitable combined treatment regimen, based on the factors cited above and his expert judgement.

In particular, the Nanobodies and polypeptides of the invention may be used in combination with other pharmaceutically active compounds or principles that are or can be used for the prevention and/or treatment of the diseases and disorders that can be prevented or treated with the fusion proteins or constructs of the invention, and as a result of which a synergistic effect may or may not be obtained.

The effectiveness of the treatment regimen used according to the invention may be determined and/or followed in any manner known per se for the disease or disorder involved, as will be clear to the clinician. The clinician will also be able, where appropriate and or a case-by-case basis, to change or modify a particular treatment regimen, so as to achieve the desired therapeutic effect, to avoid, limit or reduce unwanted side-effects, and/or to achieve an appropriate balance between achieving the desired therapeutic effect on the one hand and avoiding, limiting or reducing undesired side effects on the other hand.

Generally, the treatment regimen will be followed until the desired therapeutic effect is achieved and/or for as long as the desired therapeutic effect is to be maintained. Again, this can be determined by the clinician.

The terms and expressions which have been employed are used as terms of description and not of limitation, and there is no intention in the use of such terms and expressions of excluding any equivalents of the features shown and described or portions thereof, it being recognized that various modifications are possible within the scope of the invention.

### Experimental Part

### Example 1: Identification of serum albumin specific nanobodies

After approval of the Ethical Committee of the Faculty of Veterinary Medicine (University Ghent, Belgium), 2Ilamas (117, 118) are alternately immunized with 6 intramuscular injections at weekly interval with human serum albumin and a mixture of mouse serum albumin, cynomolgus serum albumin and baboon serum albumin, according to standard protocols.

### Library construction

When an appropriate immune response is induced in Iama, four days after the last antigen injection, a 150 ml blood sample is collected and peripheral blood lymphocytes (PBLs) are purified by a density gradient centrifugation on Ficoll-Paque™ according to the manufacturer's instructions. Next, total RNA is extracted from these cells and used as starting material for RT-PCR to amplify Nanobody encoding gene fragments. These fragments are cloned into phagemid vector pAX50. Phage is prepared according to standard methods (see for example the prior art and applications filed by applicant cited herein) and stored at 4°C for further use.

### Selection

### Selecting repertoires for binding to serum albumin.

In a first selection, human serum albumin (Sigma A-8763) is coated onto Maxisorp 96-well plates (Nunc, Wiesbaden, Germany) at 100 µg/ml overnight (ON) at room temperature (RT). Plates are blocked with 4% Marvel in PBS for 2b at RT. After 3 washes with PBST, phages are added in 4% Marvel/PBS and incubated for 1h at RT. Following extensive washing, bound phage is eluted with 0.1 M triethanolamine (TEA) and neutralized with 1M Tris-HCl pH 7.5.

### Screening for pH independent binding of Nanobodies to albumin.

### 1. Screening for pH-independent binding of Nanobodies by ELISA

To screen Nanobodies for their pH-insensitive or conditional binding to albumin, a binding ELISA is performed with two representative conditions, pH 5.8 and pH7.3 and the relative binding strength determined. Maxisorb micro titer plates (Nunc, Article No. 430341) are coated overnight at 4°C with 100 µl of a 1 µg/ml solution human serum albumin in bicarbonate buffer (50 mM, pH 9.6). After coating, the plates are washed three times with PBS containing 0.05% Tween20 (PBST) and blocked for 2 hours at room temperature (RT) with PBS containing 2% Marvel (PBSM). After the blocking step, the coated plates are washed 2 times with PBST pH 5.8, and a ten-fold dilution aliquot of each periplasmic sample in PBSM pH5.8 (100µl) is transferred to the coated plates and allowed to bind for 1 hour at RT. After sample incubation, the plates are washed five times with PBST and incubated for 1 hour at RT with 100 µl of a 1:1000 dilution of mouse anti-myc antibody in 2% PBSM. After 1 hour at RT, the plates are washed five times with PBST and incubated with 100 µl of a 1:1000 dilution of a goat anti-mouse antibody conjugated with horseradish peroxidase. After 1 hour, plates are washed five times with PBST and incubated with 100 µl of slow TMB (Pierce, Article No. 34024). After 20 minutes, the reaction is stopped with 100 µl H₂SO₄. The absorbance of each well is measured at 450 nm.

### 2. Screening of kinetic off-rate constant via surface plasmon resonance (BIAcore).

Human serum albumin is immobilized on a CM5 sensor chip surface via amine coupling using NHS/BDC for activation and ethanolamine for deactivation (Biacore amine coupling kit)

Approximately 1000RU of human serum albumin is immobilized. Experiments are performed at 25 °C. The buffers used for the pH dependent binding of Nanobodies to albumin (Biacore) are as follows: 10mM Sodium citrate (Na₃C₆H₅O₇) + 10mM Sodium phosphate (Na₂HPO₄) + 10mM Sodium Acetate (CH₃COONa) + 115mM NaCl. This mixture is brought to pH7, pH6 and pH5 by adding HCl or NaOH (dependent on the pH of the mixture measured).

Periplasmic extracts are diluted in running buffers of pH7, pH6 and pH5. The samples are injected for 1 min at a flow rate of 45ul/min over the activated and reference surfaces. Those surfaces are regenerated with a 3s pulse of glycine-HCl pH1.5 + 0.1% P20. Evaluation is done using Biacore T100 evaluation software.

### Construction of a bispecific ALB8 Nanobody

A bispecific nanobody is also generated consisting of a C-terminal anti-HSA Nanobody (ALB8), a 9 amino acid Gly/Ser linker and an N-terminal anti-IL6R Nanobody. The construct is further called IL6R202. This construct is expressed in *E.coli* as c-myc, His6-tagged proteins and subsequently purified from the culture medium by immobilized metal affinity chromatography (IMAC) and size exclusion chromotagraphy (SEC).

In all selections, enrichment is observed. The output from each selection is recloned as a pool into the expression vector pAX51. Colonies are picked and grown in 96 deep-well plates (1 ml volume) and induced by adding IPTG for Nanobody expression. Periplasmic extracts (volume: - 80 µl) are prepared according to standard methods (see for example the prior art and applications filed by applicant cited herein).

### Library evaluation by ELISA.

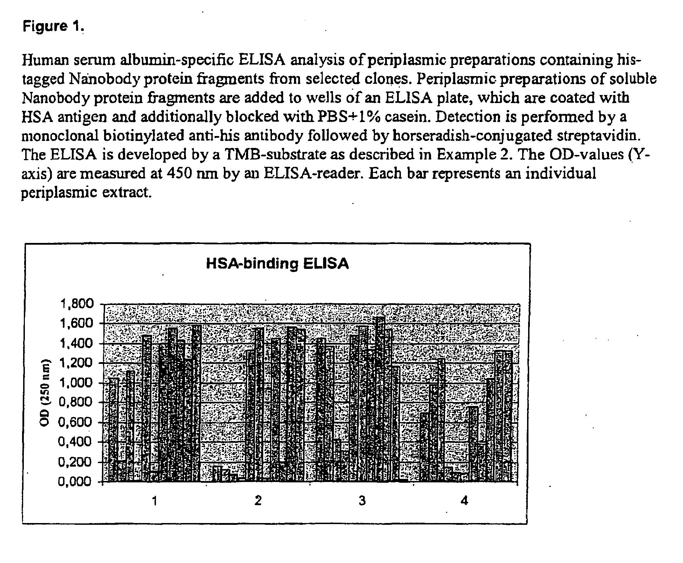

Periplasmic extracts of individual Nanobodies are screened for albumin specificity by ELISA on solid phase coated human serum albumin. Detection of Nanobody fragments bound to immobilized human serum albumin is carried out using a biotinylated mouse anti-his antibody (Serotec MCA1396B) detected with Streptavidin-HRP (DakoCytomation #P0397). The signal is developed by adding TMB substrate solution (Pierce 34021) and detected at a wavelength of 450 nm. A high hit rate of positive clones can already be obtained after panning round 1. Figure 1 is illustrative of typical ELISA results.

### Example 2: pH dependent binding of identified nanobodies to human serum albumin using surface plasmon resonance (BIAcore).

Human serum albumin is immobilized on a CM5 sensor chip surface via amine coupling using NHS/EDC for activation and ethanolamine for deactivation (Biacore amine coupling kit)

Approximately 1000RU of cynomolgus and humans serum albumin respectively is immobilized. Experiments are performed at 25 °C. The buffers used for the pH dependent binding of Nanobodies to albumin (Biacore) are as follows: 10mM Sodium citrate (Na₃C₆H₅O₇) + 10mM Sodium phosphate (Na₂HPO₄) + 10mM Sodium Acetate (CH₃C00Na) + 115mM NaCl. This mixture is brought to pH7, pH6 and pH5 by adding HCl or NaOH (dependent on the pH of the mixture measured). Standard buffer HBS-EP+ Periplasmic extracts or purified Nanobodies are diluted in running buffers of pH7, pH6 and pH5. The samples are injected for 1min at a flow rate of 45ul/min over the activated and reference surfaces. Those surfaces are regenerated with a 3s pulse of glycine-HCl pH1.5 + 0.1 % P20. Evaluation is done using Biacore T100 evaluation software.

The off rate of different Nanobodies at pH7 and pH5 is documented in Table 1. The majority of the Nanobodies (4A2, 4A6, 4B5, 4B6, 4B8, 4C3, 4C4, 4C5, 4C8, 4C9, 4D3, 4D4, 4D7 and 4D10 have a faster off rate at pH 5 compared with pH 7. Nanobody 4A9 has a slower off-rate at pH 5 compared to pH 7. For other Nanobodies including IL6R202, Alb-8, 4C11, 4B1, 4B10 and 4D5, binding to antigen does not change at different pH.

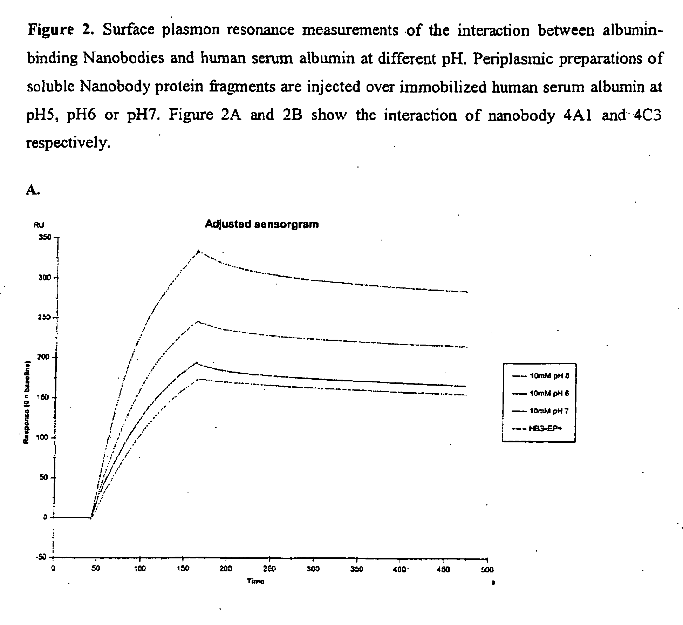

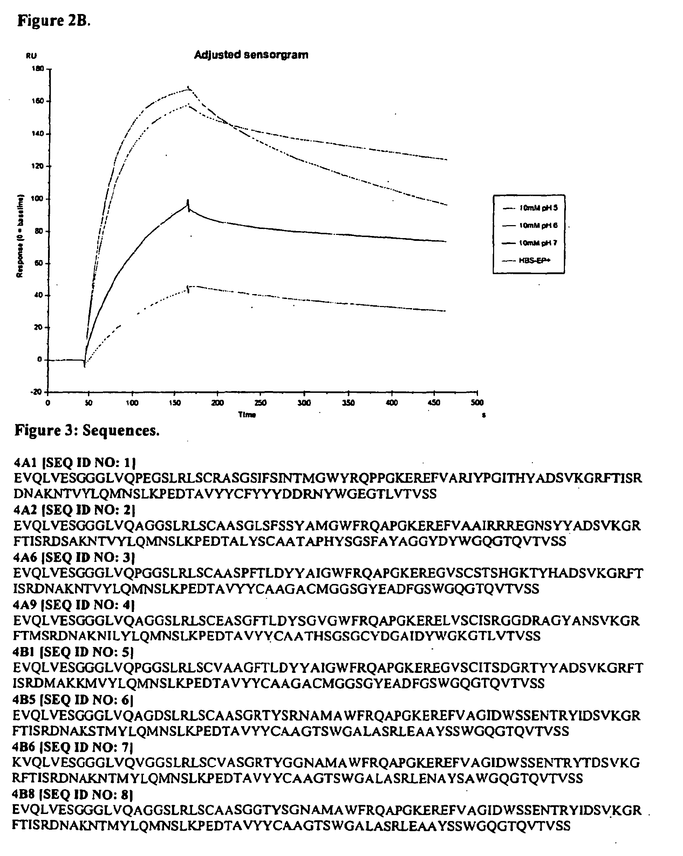

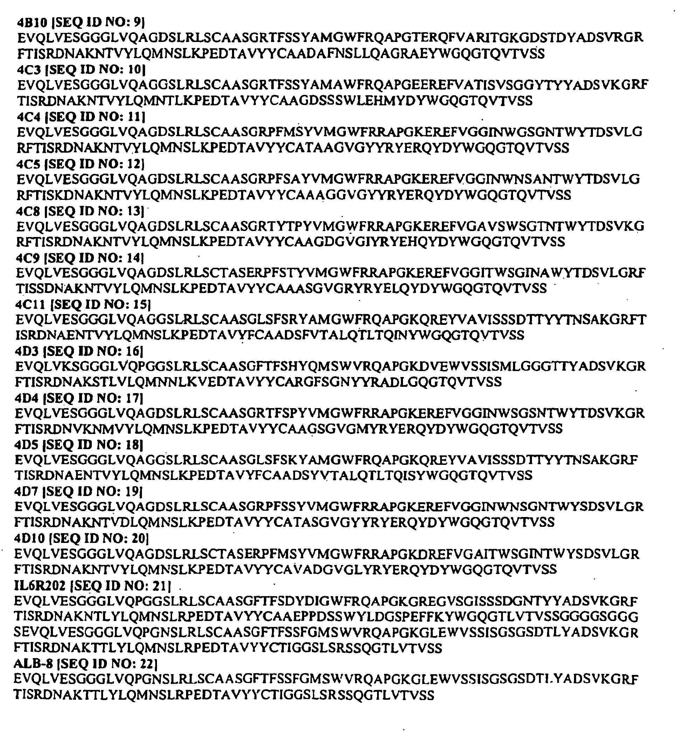

The sensorgram of representative clones are shown in Figure 2a and 2B.

### Example 2a: Fusion of albumin-binding Nanobody ALB8 to a Nanobody directed against therapeutic target IL6R (IL6R202) does not impact on its pH independent binding to human or cynomolgus serum albumin.

The pH-insensitive binding properties of IL6R202 are evaluated via surface plasmon resonance (BIAcore). Human and cynomolgus serum albumin is immobilized on a CM5 sensor chip surface via amine coupling using NHS/EDC for activation and ethanolamine for deactivation (Biacore amine coupling kit).

Approximately 1000RU of cynomolgus and humans serum albumin respectively is immobilized. Experiments are performed at 25 °C. The buffers used for the pH dependent binding of Nanobodies to albumin (Biacore) are as follows: 10mM Sodium citrate (Na₃C₆H₅O₇) + 10mM Sodium phosphate (Na₂HPO₄ + 10mM Sodium Acetate (CH₃C00Na) + 115M NaCl. This mixture is brought to pH7, pH6 and pH5 by adding HCl or NaOH (dependent on the pH of the mixture measured).

Periplasmic extracts or purified Nanobodies are diluted in running buffers of pH7, pH6 and pH5. The samples are injected for 1min at a flow rate of 45ul/min over the activated and reference surfaces. Those surfaces are regenerated with a 3s pulse of glycine-HCl pH1.5 + 0.1% P20. Evaluation is done using Biacore T100 evaluation software. The binding characteristics of IL6R202 are shown below. When ALB8 is incorporated into a the bispecific nanobody format IL6R202, the pH-independent binding characteristics are not different compared to its pH-independent binding characteristics as monovalent Nanobody. Moreover does the ALB8 within the bispecific construct display cross-reactivity to the cynomolgus serum albumin and with similar binding characteristics.

### Binding kinetics of ALB8 to human serum albumin.

| | **kon (1/Ms) at pH7** | **koff (1/s) at pH 7** | **koff (1/s) at pH 5** |
|---|---|---|---|
| **Human scrum albumin** | 3.37E05 | 2,97E-03 | 2,78E-03 |

### Binding kinetics of IL-6R202 to human and cyno serum albumin

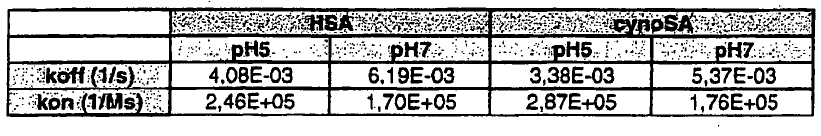

### Example 3: Pharmacokinetic profile in cynomolgus monkey

A Nanobody (Nanobody is called: IL6R202) is constructed to a bivalent construct using an humanized anti-IL6R building block and a humanized anti-serum albumin building block (humanized anti-serum albumin building block = Alb-8). A 9-amino acid GlySer linker is used to link the different building blocks. This construct is expressed in E. coli and purified using ProtA followed by size exclusion chromatography (SEC). A pharmacokinetic study of IL6R202 (with a k-off rate independent of pH - see above) is conducted in cynomolgus monkeys. IL6R202 is administered intravenously by bolus injection (1.0 ml/kg, approximately 30 sec) in the vena cephalica of the left or right arm to obtain a dose of 2.0 mg/kg. Table 2 summarizes the dosing regimen for all monkeys.

| **Compound** | **Route** | **Animal** | **Animal ID** | **Dose Volume (ml/kg)** | **Dose (mg/kg)** |
|---|---|---|---|---|---|
| IL6R202 | Iv (bolus) | Cynomolgus Monkey | 3 m | 1.0 | 2.0 |
| | Iv (bolus) | Cynomolgus Monkey | 4 f | 1.0 | 2.0 |

### IL6R302 concentration in the plasma samples is determined as follows:

Maxisorb micro titer plates (Nunc, Article No. 430341) are coated overnight at 4°C with 100 µl of a 5 µg/ml solution of 12B2-GS9-12B2 (B2#1302nr4.3.9) in bicarbonate buffer (50 mM, pH 9.6). After coating, the plates are washed three times with PBS containing 0. 1% Tween20 and blocked for 2 hours at room temperature (RT) with PBS containing 1 % casein (250 (µl/well). Plasma samples and serial dilutions of Nanobody-standards (spiked in 100% pooled blank cynomolgus plasma) are diluted in PBS in a separate non-coated plate (Nunc, Article No. 249944) to obtain the desired concentration/dilution in a final sample matrix consisting of 10% pooled cynomolgus plasma in PBS. All pre-dilutions are incubated for 30 minutes at RT in the non-coated plate. After the blocking step, the coated plates are washed three times (PBS containing 0.1% Tween20), and an aliquot of each sample dilution (100µl) is transferred to the coated plates and allowed to bind for 1 hour at RT. After sample incubation, the plates are washed three times (PBS containing 0.1% Tween20) and incubated for 1 hour at RT with 100 µl of a 100 ng/ml solution of sIL6R in PBS (Peprotech, Article No. 20006R). After 1 hour at RT, the plates are washed three times (PBS containing 0.1% Tween20) and incubated with 100 µl of a 250 ng/ml solution of a biotinylated polyclonal anti-IL6R antibody in PBS containing 1% casein (R&D systems, Article No. BAF227). After incubation for 30 minutes (RT), plates are washed three times (PBS containing 0.1% Tween20) and incubated for 30 minutes (RT) with 100 µl of a 1/5000 dilution (in PBS containing 1% casein) of streptavidine conjugated with horseradish peroxidase (DaktoCytomation, Article No. P0397). After 30 minutes, plates are washed three times (PBS containing 0.1% Tween20) and incubated with 100 µl of slow TMB (Pierce, Article No. 34024). After 20 minutes, the reaction is stopped with 100 µl HCl (1N). The absorbance of each well is measured at 450 nm (Tecan Sunrise spectrophotometer), and corrected for absorbance at 620 nm. This assay measures free Nanobody as well as Nanobodies bound to sIL6R and/or cynomolgus serum albumin. Concentration in each plasma sample is determined based on a sigmoidal standard curve with variable slope of the respective Nanobody. The LLOQ and ULOQ of IL6R202 are 7.00 ng/ml.

Each individual plasma sample is analyzed in two independent assays and an average plasma concentration is calculated for pharmacokinetic data analysis.

Basic pharmacokinetic parameters of IL6R202 after a single intravenous administration at 2.00 mg/kg in the male and female cynomolgus monkey are listed in Table 3. All parameters are calculated with two-compartmental modeling, with elimination from the central compartment.

Determined half life of IL6R202 is 6.8 +/- 0.226 days which is very similar to the half life of Cynomolgus Monkey-serum albumin in Cynomolgus Monkey.

**Table 3: Basic pharmacokinetic parameters¹ of IL6R202 after a single intravenous administration at 2.00 mg/kg in the male and female Cynomolgus Monkey.**

| | Monkey 3m | Monkey 4f | Mean | SD | CV(%) |
|---|---|---|---|---|---|
| C₍₀₎ (µg/ml) | 57.6 | 56.5 | 57.1 ± | 0.778 | 1.36 |
| Vₛₛ (mL/kg) | 65.1 | 60.6 | 62.9 ± | 3.18 | 5.06 |
| V_{z} (mL/kg) | 70.0 | 64.6 | 67.3 ± | 3.82 | 5.67 |
| V_{c} (mL/kg) | 34.7 | 35.4 | 35.1 ± | 0.495 | 1.41 |
| Vₜ (mL/kg) | 30.4 | 25.2 | 27.8 ± | 3.68 | 13.2 |
| CL (mL/day/kg) | 6.97 | 6.74 | 6.86 ± | 0.163 | 2.37 |
| CL_{d} (mL/day/kg) | 22.1 | 19.1 | 20.6 ± | 2.12 | 10.3 |
| t_{½} (day) | 6.96 | 6.64 | 6.80 ± | 0.226 | 3.33 |
| MRT (day) | 9.35 | 8.99 | 9.17 | 0.255 | 2.78 |
| AUC_{inf}(µg•day/ml) | 287 | 297 | 292 ± | 7.07 | 2.42 |
| AUC_{inf}/D | 0.144 | 0.148 | ± | | |
| (kg•day/ml) | | | 0.146 | 0.00283 | 1.94 |

| | | | | | |
|---|---|---|---|---|---|
| ¹ All parameters were calculated with two-compartmental modeling | | | | | |

**Table 1. Off rate (determined by Biacore) of different Nanobodies® at pH7 and pH5 is documented**

| **Nanobody** | **kd (1/s) at pH 7** | **kd (1/s) at pH 5** | **Ratio pH7/pH5** |
|---|---|---|---|
| 4D10 | 5,23E-04 | 3,41 E-03 | 6,52 |
| 4A6 | 1,73E-03 | 9,99E-03 | 5,77 |
| 4C9 | 4.41E-04 | 1.71E-03 | 3.88 |
| 4A2 | 6.42E-03 | 2,27E-02 | 3,54 |
| 4C8 | 6,24E-04 | 2,09E-03 | 3.35 |
| 4C3 | 1,12E-03 | 3,75E-03 | 3,35 |
| 4B6 | 3,68E-04 | 1,19E-03 | 3,23 |
| 4D4 | 6,02E-03 | 1,66E-02 | 2,76 |
| 4C5 | 5.41 E-04 | 1,32E-03 | 2,44 |
| 4B8 | 7,41E-04 | 1.80E-03 | 2,43 |
| 4C4 | 4,99E-04 | 1,21E-03 | 2,42 |
| 4D3 | 5.65E-03 | 1,37E-02 | 2,42 |
| 4D7 | 6,53E-04 | 1,58E-03 | 2,42 |
| 4B5 | 1,74E-03 | 4,03E-03 | 2,32 |
| 4D5 | 2,04E-02 | 2,63E-02 | 1,29 |
| 4C11 | 2,63E-02 | 3,12E-02 | 1,19 |
| 4B1 | 8.75E-03 | 7,73E-03 | 0,88 |
| 4B10 | 4.99E-02 | 4,34E-02 | 0,87 |
| 4A9 | 1,30E-02 | 7,01E-03 | 0,54 |
| Alb8 | 2,97E-03 | 2,78E-03 | 1.07 |
| IL-6R202 | 4.08E-03 | 6.19E-03 | 1,52 |

## Claims

1. Immunoglobulin variable domain sequence that binds to a serum protein chosen from the group consisting of serum albumin, immunoglobulin or transferrin,
wherein said immunoglobulin variable domain sequence binds to the serum protein at a pH value in the range of 6.5 to 5.5 with an association constant (K_{A}) that is at least 50% of the association constant (K_{A}) with which said immunoglobulin variable domain sequence binds to the same serum protein at a pH in the range of 7.2 to 7.4,
wherein said immunoglobulin variable domain sequence is a domain antibody, dAb, single domain antibody or Nanobody.

2. Immunoglobulin variable domain sequence according to claim 1, that can bind to said serum protein in such a way that, when the immunoglobulin variable domain sequence is bound to said serum protein molecule, the half-life of the said serum protein molecule is not reduced.

3. Immunoglobulin variable domain sequence according to any of the preceding claims, that binds to a serum protein that can bind to FcRn.

4. Immunoglobulin variable domain sequence according to any of the preceding claims, that binds to a serum protein of at least one species of primate in such a way that, when the immunoglobulin variable domain sequence is bound to said serum protein in said primate, said immunoglobulin variable domain sequence exhibits a serum half-life of at least 50% of the natural serum half-life of said serum protein in said primate.

5. Immunoglobulin variable domain sequence according to claim 4, wherein said immunoglobulin variable domain sequence exhibits a serum half-life of at least 7 days.

6. Immunoglobulin variable domain sequence according to any preceding claim, wherein said immunoglobulin variable domain sequence is a fully human, humanized, camelid, camelized human or humanized camelid sequence.

7. Immunoglobulin variable domain sequence according to any preceding claim, which is selected from any one of SEQ ID No. 4, SEQ ID No. 5, SEQ ID No. 9, SEQ ID No. 15, SEQ ID No. 18, SEQ ID No. 21 and SEQ ID No. 22.

8. Compound comprising the immunoglobulin variable domain sequence of any one of claims 1 to 7 and at least one therapeutic moiety.

9. Compound according to claim 8, in which the therapeutic moiety comprises a domain antibody or a Nanobody.

10. Nucleotide sequence or nucleic acid that encodes the immunoglobulin variable domain sequence according to any of claims 1 to 7, or the immunoglobulin variable domain sequence of a compound according to claim 8 or 9.

11. Hosts or host cells that contain a nucleotide sequence or nucleic acid according to claim 10, and/or that express the immunoglobulin variable domain sequence according to any of claims 1 to 7, or the immunoglobulin variable domain sequence of a compound according to any one of claims 8 or 9.

12. Method for preparing the immunoglobulin variable domain sequence according to any of claims 1 to 7, or the immunoglobulin variable domain sequence of a compound according to claim 8 or 9, which method comprises cultivating or maintaining a host cell according to claim 11 under conditions such that said host cell produces or expresses the immunoglobulin variable domain sequence according to any of claims 1 to 7, or the immunoglobulin variable domain sequence of a compound according to claim 8 or 9, and further comprises isolating the immunoglobulin variable domain sequence according to any of claims 1 to 7, or the amino acid variable domain sequence of a compound according to claim 8 or 9 so produced.

13. Pharmaceutical composition comprising a compound of claim 8 or 9 and at least one pharmaceutically acceptable carrier, diluent or excipient, wherein said pharmaceutical composition is suitable for administration to a primate at interval(s) of at least 50% of the natural half-life of said serum protein in said primate.

14. Use of any of the immunoglobulin variable domain sequence according to any one of claims 1 to 7, or the compound according to claim 8 or 9 for the manufacture of a medicament for administration to a primate, wherein said medicament is administered at interval(s) of at least 50% of the natural half-life of said serum protein in said primate.

15. Use according to claim 14, wherein the medicament is administered at interval(s) of at least 7 days.

16. Use of an immunoglobulin variable domain sequence according to any one of claims 1 to 7 for increasing the half life of a therapeutic moiety.

17. Method for extending or increasing the serum half-life of a biological therapeutic comprising contacting the therapeutic with an immunoglobulin variable domain sequence according to any one of claims 1 to 7.

18. The method according to claim 17, wherein the biological therapeutic is a peptide or polypeptide, and wherein the step of contacting the therapeutic comprises preparing a fusion protein by linking the peptide or polypeptide with the immunoglobulin variable domain sequence according to any one of claims 1 to 7.

## Patentansprüche

1. Sequenz einer variablen Domäne eines Immunglobulins, die an ein Serumprotein ausgewählt aus der Gruppe bestehend aus Serumalbumin, Immunglobulin oder Transferrin bindet,
wobei die Sequenz einer variablen Domäne eines Immunglobulins an das Serumprotein bei einem pH-Wert im Bereich von 6,5 bis 5,5 mit einer Assoziationskonstante (K_{A}) bindet, die mindestens 50 % der Assoziationskonstante (K_{A}) beträgt, mit der die Sequenz einer variablen Domäne eines Immunglobulins an das gleiche Serumprotein bei einem pH im Bereich von 7,2 bis 7,4 bindet,
wobei die Sequenz einer variablen Domäne eines Immunglobulins ein Domänenantikörper, dAb, Einzeldomänenantikörper oder Nanobody ist.

2. Sequenz einer variablen Domäne eines Immunglobulins gemäß Anspruch 1, die an das Serumprotein so binden kann, dass, wenn die Sequenz einer variablen Domäne eines Immunglobulins an das Serumproteinmolekül gebunden ist, die Halbwertszeit des Serumproteinmoleküls nicht vermindert ist.

3. Sequenz einer variablen Domäne eines Immunglobulins gemäß einem der vorhergehenden Ansprüche, die an ein Serumprotein bindet, das an FcRn binden kann.

4. Sequenz einer variablen Domäne eines Immunglobulins gemäß einem der vorhergehenden Ansprüche, die an ein Serumprotein von mindestens einer Primatenspezies so bindet, dass, wenn die Sequenz einer variablen Domäne eines Immunglobulins an das Serumprotein in dem Primaten gebunden ist, die Sequenz einer variablen Domäne eines Immunglobulins eine Serumhalbwertszeit von mindestens 50 % der natürlichen Serumhalbwertszeit des Serumproteins in dem Primaten zeigt.

5. Sequenz einer variablen Domäne eines Immunglobulins gemäß Anspruch 4, wobei die Sequenz einer variablen Domäne eines Immunglobulins eine Serumhalbwertszeit von mindestens 7 Tagen zeigt.

6. Sequenz einer variablen Domäne eines Immunglobulins gemäß einem vorhergehenden Anspruch, wobei die Sequenz einer variablen Domäne eines Immunglobulins eine vollhumane, humanisierte, Cameliden-, camelisierte humane oder humanisierte Camelidensequenz ist.

7. Sequenz einer variablen Domäne eines Immunglobulins gemäß einem vorhergehenden Anspruch, die aus einer von SEQ ID NO: 4, SEQ ID NO: 5, SEQ ID NO: 9, SEQ ID NO: 15, SEQ ID NO: 18, SEQ ID NO: 21 und SEQ ID NO: 22 ausgewählt wird.

8. Verbindung, umfassend die Sequenz einer variablen Domäne eines Immunglobulins gemäß einem der Ansprüche 1 bis 7 und mindestens eine therapeutische Gruppe.

9. Verbindung gemäß Anspruch 8, bei welcher die therapeutische Gruppe einen Domänenantikörper oder Nanobody umfasst.

10. Nukleotidsequenz oder Nukleinsäure, die die Sequenz einer variablen Domäne eines Immunglobulins gemäß einem der Ansprüche 1 bis 7 oder die Sequenz einer variablen Domäne eines Immunglobulins einer Verbindung gemäß Anspruch 8 oder 9 codiert.

11. Wirte oder Wirtszellen, die eine Nukleotidsequenz oder Nukleinsäure gemäß Anspruch 10 enthalten, und/oder die die Sequenz einer variablen Domäne eines Immunglobulins gemäß einem der Ansprüche 1 bis 7, oder die Sequenz einer variablen Domäne eines Immunglobulins einer Verbindung gemäß einem der Ansprüche 8 oder 9 exprimieren.

12. Verfahren zum Herstellen der Sequenz einer variablen Domäne eines Immunglobulins gemäß einem der Ansprüche 1 bis 7, oder der Sequenz einer variablen Domäne eines Immunglobulins einer Verbindung gemäß Anspruch 8 oder 9, wobei das Verfahren das Kultivieren oder Erhalten einer Wirtszelle gemäß Anspruch 11 unter Bedingungen umfaßt, bei denen die Wirtszelle die Sequenz einer variablen Domäne eines Immunglobulins gemäß einem der Ansprüche 1 bis 7, oder die Sequenz einer variablen Domäne eines Immunglobulins einer Verbindung gemäß Anspruch 8 oder 9 herstellt oder exprimiert, sowie weiterhin das Isolieren der Sequenz einer variablen Domäne eines Immunglobulins gemäß einem der Ansprüche 1 bis 7 oder der Aminosäuresequenz einer variablen Domäne einer Verbindung gemäß Anspruch 8 oder 9, die auf diese Weise hergestellt wurde, umfasst.

13. Pharmazeutische Zusammensetzung umfassend eine Verbindung gemäß Anspruch 8 oder 9 und mindestens ein(en) pharmazeutisch annehmbaren/s Träger, Verdünnungsmittel oder Hilfsstoff, wobei die pharmazeutische Zusammensetzung für die Verabreichung an einen Primaten im Abstand/in Abständen von mindestens 50 % der natürlichen Halbwertszeit des Serumproteins in diesem Primaten geeignet ist.

14. Verwendung der Sequenz einer variablen Domäne eines Immunglobulins gemäß einem der Ansprüche 1 bis 7, oder der Verbindung gemäß Anspruch 8 oder 9, zur Herstellung eines Arzneimittels zur Verabreichung an einen Primaten, wobei das Arzneimittel im Abstand/in Abständen von mindestens 50 % der natürlichen Halbwertszeit des Serumproteins in diesem Primaten verabreicht wird.

15. Verwendung gemäß Anspruch 14, wobei das Arzneimittel im Abstand/in Abständen von mindestens 7 Tagen verabreicht wird.

16. Verwendung der Sequenz einer variablen Domäne eines Immunglobulins gemäß einem der Ansprüche 1 bis 7 zur Herstellung eines Arzneimittels zum Erhöhen der Serumhalbwertszeit einer therapeutischen Gruppe.

17. Verfahren zur Herstellung eines Arzneimittels zum Erweitern oder Erhöhen der Serumhalbwertszeit eines biologischen Therapeutikums, umfassend das In-Kontakt-Bringen des Therapeutikums mit der Sequenz einer variablen Domäne eines Immunglobulins gemäß einem der Ansprüche 1 bis 7.

18. Verfahren gemäß Anspruch 17, wobei das biologische Therapeutikum ein Peptid oder Polypeptid ist, und wobei der Schritt des In-Kontakt-Bringens des Therapeutikums das Herstellen eines Fusionsproteins durch Verknüpfen des Peptids oder Polypeptids mit der Sequenz einer variablen Domäne eines Immunglobulins gemäß einem der Ansprüche 1 bis 7 umfasst.

## Revendications

1. Séquence de domaine variable d'une immunoglobuline qui se lie à une protéine sérique choisie dans le groupe constitué par la sérum albumine, une immunoglobuline ou la transferrine,
dans laquelle ladite séquence de domaine variable d'immunoglobuline se lie à la protéine sérique à une valeur de pH dans la gamme de 6,5 à 5,5 avec une constante d'association (K_{A}) qui est au moins 50% de la constante d'association (K_{A}) avec laquelle ladite séquence de domaine variable d'immunoglobuline se lie à la même protéine sérique à un pH dans la gamme de 7,2 à 7,4,
dans laquelle ladite séquence de domaine variable d'immunoglobuline est un anticorps à domaine, un dAb, un anticorps à domaine unique ou un Nanobody.

2. Séquence de domaine variable d'une immunoglobuline selon la revendication 1, qui peut se lier à ladite protéine sérique de telle manière que, lorsque la séquence de domaine variable d'immunoglobuline est liée à ladite molécule de protéine sérique, la demi-vie de ladite molécule de protéine sérique n'est pas réduite.

3. Séquence de domaine variable d'une immunoglobuline selon l'une quelconque des revendications précédentes, qui se lie à une protéine sérique qui peut se lier au FcRn.

4. Séquence de domaine variable d'une immunoglobuline selon l'une quelconque des revendications précédentes, qui se lie à une protéine sérique d'au moins une espèce de primate de telle manière que, lorsque la séquence de domaine variable d'immunoglobuline est liée à ladite protéine sérique chez ledit primate, ladite séquence de domaine variable d'immunoglobuline présente une demi-vie sérique d'au moins 50% de la demi-vie sérique naturelle de ladite protéine sérique chez ledit primate.

5. Séquence de domaine variable d'une immunoglobuline selon la revendication 4, dans laquelle ladite séquence de domaine variable d'immunoglobuline présente une demi-vie sérique d'au moins 7 jours.

6. Séquence de domaine variable d'une immunoglobuline selon l'une quelconque des revendications précédentes, dans laquelle ladite séquence de domaine variable d'immunoglobuline est une séquence entièrement humaine, humanisée, de camélidé, humaine camélisée ou de camélidé humanisée.

7. Séquence de domaine variable d'une immunoglobuline selon l'une quelconque des revendications précédentes, qui est choisie parmi l'une quelconque de SEQ ID No. 4, SEQ ID No. 5, SEQ ID No. 9, SEQ ID No. 15, SEQ ID No. 18, SEQ ID No. 21 et SEQ ID No. 22.

8. Composé comprenant la séquence de domaine variable d'une immunoglobuline selon l'une quelconque des revendications 1 à 7 et au moins une fraction thérapeutique.

9. Composé selon la revendication 8, dans lequel la fraction thérapeutique comprend un anticorps à domaine ou un Nanobody.

10. Séquence nucléotidique ou acide nucléique qui code pour la séquence de domaine variable d'immunoglobuline selon l'une quelconque des revendications 1 à 7, ou la séquence de domaine variable d'immunoglobuline d'un composé selon la revendication 8 ou 9.

11. Hôtes ou cellules hôtes qui contiennent une séquence nucléotidique ou un acide nucléique selon la revendication 10, et/ou qui expriment la séquence de domaine variable d'immunoglobuline selon l'une quelconque des revendications 1 à 7, ou la séquence de domaine variable d'immunoglobuline d'un composé selon l'une quelconque des revendications 8 ou 9.

12. Procédé pour préparer la séquence de domaine variable d'une immunoglobuline selon l'une quelconque des revendications 1 à 7, ou la séquence de domaine variable d'immunoglobuline d'un composé selon la revendication 8 ou 9, lequel procédé comprend la culture ou le maintien d'une cellule hôte selon la revendication 11 dans des conditions telles que ladite cellule hôte produit ou exprime la séquence de domaine variable selon l'une quelconque des revendications 1 à 7, ou la séquence de domaine variable d'immunoglobuline d'un composé selon la revendication 8 ou 9, et comprend en outre l'isolement de la séquence de domaine variable d'immunoglobuline selon l'une quelconque des revendications 1 à 7, ou la séquence d'acides aminés de domaine variable d'un composé selon la revendication 8 ou 9 ainsi produite.

13. Composition pharmaceutique comprenant un composé selon la revendication 8 ou 9 et au moins un support, un diluant ou un excipient pharmaceutiquement acceptable, dans laquelle ladite composition pharmaceutique est adaptée à l'administration à un primate à un(des)intervalle(s) d'au moins 50% de la demi-vie naturelle de la ladite protéine sérique chez ledit primate.

14. Utilisation de l'une quelconque de la séquence de domaine variable d'immunoglobuline selon l'une quelconque des revendications 1 à 7, ou du composé selon la revendication 8 ou 9 pour la fabrication d'un médicament destiné à l'administration à un primate, dans laquelle ledit médicament est administré à un(des)intervalle(s) d'au moins 50% de la demi-vie naturelle de la ladite protéine sérique chez ledit primate.

15. Utilisation selon la revendication 14, dans laquelle le médicament est administré à un (des)intervalle(s) d'au moins 7 jours.

16. Utilisation d'une séquence de domaine variable d'immunoglobuline selon l'une quelconque des revendications 1 à 7 pour la préparation d'un médicament destiné à augmenter la demi-vie sérique d'une fraction thérapeutique.

17. Procédé pour la préparation d'un médicament destiné à prolonger ou augmenter la demi-vie sérique d'un agent thérapeutique biologique comprenant la mise en contact de l'agent thérapeutique avec une séquence de domaine variable d'immunoglobuline selon l'une quelconque des revendications 1 à 7.

18. Procédé selon la revendication 17, dans lequel l'agent thérapeutique biologique est un peptide ou un polypeptide, et dans lequel l'étape de mise en contact de l'agent thérapeutique comprend la préparation d'une protéine de fusion par liaison du peptide ou polypeptide à la séquence de domaine variable d'immunoglobuline selon l'une quelconque des revendications 1 à 7.
